(19) Europäisches Patentamt
European Patent Office
Office européen des brevets

(11) EP 1 802 197 B1

(12) **EUROPÄISCHE PATENTSCHRIFT**

(45) Veröffentlichungstag und Bekanntmachung des Hinweises auf die Patenterteilung:
**28.02.2018 Patentblatt 2018/09**

(21) Anmeldenummer: **05792754.3**

(22) Anmeldetag: **29.09.2005**

(51) Int Cl.:
***A01N 43/56*** *(2006.01)* ***A01N 61/00*** *(2006.01)*
***A01P 3/00*** *(2006.01)*

(86) Internationale Anmeldenummer:
**PCT/EP2005/010522**

(87) Internationale Veröffentlichungsnummer:
**WO 2006/040016 (20.04.2006 Gazette 2006/16)**

(54) **FUNGIZIDE WIRKSTOFFKOMBINATIONEN**

FUNGICIDAL COMBINATIONS OF ACTIVE INGREDIENTS

COMBINAISONS DE SUBSTANCES ACTIVES FONGICIDES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **08.10.2004 DE 102004049041**

(43) Veröffentlichungstag der Anmeldung:
**04.07.2007 Patentblatt 2007/27**

(73) Patentinhaber: **Bayer Intellectual Property GmbH**
**40789 Monheim am Rhein (DE)**

(72) Erfinder:
- **FISCHER, Reiner**
  **40789 Monheim (DE)**
- **DAHMEN, Peter**
  **41470 Neuss (DE)**
- **WACHENDORFF-NEUMANN, Ulrike**
  **56566 Neuwied (DE)**

(74) Vertreter: **Bosser, Isabelle**
**Bayer S.A.S.**
**Patents & Licensing Department**
**14 impasse Pierre Baizet**
**CS99163**
**69263 Lyon Cedex 09 (FR)**

(56) Entgegenhaltungen:
- **DATABASE AGRICOLA [Online] SZERSZEN, J.B. ET AL: "Interactions between and among grain sorghum, sorghum downy mildew, and the seed herbicide antidotes Concep II, Concep, and Screen." XP002355580 gefunden im STN Database accession no. 89:39538 & PHYTOPATHOLOGY, Bd. 78, Nr. 12, Dezember 1998 (1998-12), Seiten 1648-1655,**
- **DATABASE CAPLUS [Online] CHEMICAL ABSTRACTS SERVICE, COLUMBUS, OHIO, US; BERGMANN, HANS ET AL: "Ethanolamine antidotes for plant growth inhibitors and fungicides , on crops." XP002355581 gefunden im STN Database accession no. 1991:77036 & DD 277 829 A1 (AKADEMIE DER LANDWIRTSCHAFTSWISSENSCHAFTEN DER DDR, FORSCHUNGSZENTRUM) 18. April 1990 (1990-04-18)**
- **DATABASE CAPLUS [Online] CHEMICAL ABSTRACTS SERVICE, COLUMBUS, OHIO, US; "Magnesium hydroxide as safening agent for cupric hydroxide fungicide" XP002355582 gefunden im STN Database accession no. 1982:522066 & JP 57 112311 A2 (HOKKO CHEMICAL INDUSTRY CO., LTD., JAPAN) 13. Juli 1982 (1982-07-13)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 802 197 B1

- **DATABASE CAPLUS [Online] CHEMICAL ABSTRACTS SERVICE, COLUMBUS, OHIO, US; BURCHILL, R. T. ET AL: "Apple spray programs used at East Malling to control scab and mildew" XP002355583 gefunden im STN Database accession no. 1976:554950 & REPORT - EAST MALLING RESEARCH STATION (MAIDSTONE, ENGLAND) 161-5 CODEN: EMRSAV; ISSN: 0306-6398, 1974,**
- **DATABASE CAPLUS [Online] CHEMICAL ABSTRACTS SERVICE, COLUMBUS, OHIO, US; "Safening agents for ethylenebisdithiocarbamate fungicides" XP002355584 gefunden im STN Database accession no. 1985:591458 & JP 60 123405 A2 (TOKYO ORGANIC CHEMICAL INDUSTRIES, LTD., JAPAN) 2. Juli 1985 (1985-07-02)**

Bemerkungen:

Die Akte enthält technische Angaben, die nach dem Eingang der Anmeldung eingereicht wurden und die nicht in dieser Patentschrift enthalten sind.

**Beschreibung**

Die Erfindung betrifft die Verwendung von Safenern zur Wirkungssteigerung von Fungiziden, Kombinationen von Fungiziden und Safenern und deren Verwendung zur Bekämpfung von unerwünschten Mikroorganismen.

Zahlreiche Triazolderivate, Strobilurine, Anilinderivate, Dicarboximide, Carboxamide und andere chemische Verbindungen werden bereits zur Bekämpfung von unerwünschten Mikroorganismen eingesetzt (siehe z.B. The Pesticide Manual, 13th Edition, Farnham, 2003).

Szerszen, J.B. et al. offenbart, dass ein Concept II Safener das Auftreten von falschem Mehltau erhöht, und er eine fungizide Wirkung auf Mikroorganismen hat, die natürlich mit Oosporen von P.sorghi assoziiert sind. (Phytopatholy, Ecology and Epidemiology, Bd.78, Nr.12, Dezember 1998).

Mittel zur Erhöhung der Toleranz von Kulturpflanzen gegenüber chemischen Stressoren oder gegenüber ungünstigen Nebenwirkungen bekannter Wachstumsregulatoren und Fungizide sind bekannt (DD277829 ; JP57112311 ; JP60123405 ; Burchill, R.T. ET Al: "Apple spray programs used at East Malling to control scab and mildew", Report, East Malling Research Station (Maidstone, England) (1974) 161-5).

Da sich aber die ökologischen und ökonomischen Anforderungen an moderne Fungizide laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Fungizide zu entwickeln, die zumindest in Teilbereichen Vorteile gegenüber den bekannten aufweisen.

Es wurde nun überraschend gefunden, dass sich sogenannte Safener, d.h. die Kulturpflanzenverträglichkeit gegenüber Herbiziden verbessernde Verbindungen, dazu eignen, die Wirkung von Fungiziden gegenüber unerwünschten Mikroorganismen, insbesondere phytopathogenen Pilzen, zu steigern.

Dies ist umso erstaunlicher, als die Safener allein in der Regel keinerlei Wirkung gegen die unerwünschten Mikroorganismen zeigen.

Gegenstand der Erfindung ist daher die Verwendung von die Kulturpflanzenverträglichkeit gegenüber Herbiziden verbessernden Verbindungen (Safenern) zur Steigerung der mikrobiziden, insbesondere fungiziden Wirkung von Fungiziden.

Gegenstand der Erfindung ist daher ein fungizides Mittel, enthaltend

(a) ein Fungizid ausgewählt aus der Gruppe bestehend aus Trifloxystrobin, Fluoxastrobin und Prothioconazole, und einen Safener ausgewählt aus der Gruppe bestehend aus Mefenpyrdiethyl und Isoxadifen-ethyl, oder

(b) ein Fungizid ausgewählt aus der Gruppe bestehend aus Azoxystrobin und Pyraclostrobin und einen Safener ausgewählt aus der Gruppe bestehend aus Mefenpyr-diethyl, Isoxadifen-ethyl, Cloquintocet-mexyl und N-[[4-[(cyclopropylamino)carbonyl]-phenyl]sulfonyl]-2-methoxybenzamid (= I-e-5)

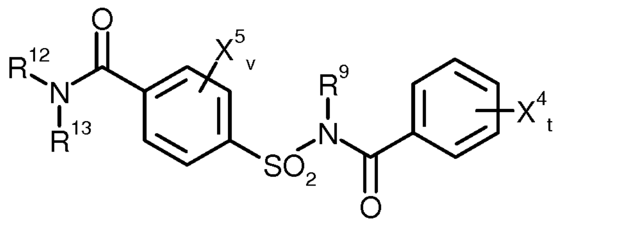

(I-e)

| Nr. | $R^9$ | $R^{12}$ | $R^{13}$ | $X^4_t$ | $X^5_v$ |
|---|---|---|---|---|---|
| I-e-5 | H | H | 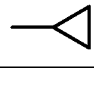 | 2-$OCH_3$ | - |

oder

(c) das Fungizid Tebuconazole und den Safener Mefenpyr-diethyl.

Die erfindungsgemäßen Kombinationen können als solche oder in ihren Formulierungen auch in Mischung mit weiteren bekannten Fungiziden, vorzugsweise aus den Gruppen 2 bis 24, Bakteriziden, Akariziden, Nematiziden oder Insektiziden verwendet werden - insbesondere bei der Behandlung von Saatgut -, um so z.B. das Wirkungsspektrum zu verbreitern oder Resistenzentwicklungen vorzubeugen.

Bei den erfindungsgemäß eingesetzten Fungiziden handelt es sich im Allgemeinen um eine oder mehrere Verbindungen aus den Gruppen (2) bis (24):

Gruppe (2) Strobilurine der allgemeinen Formel (II)

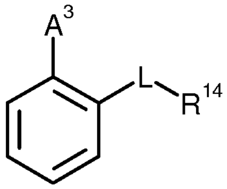

(II)

in welcher

$A^3$ für eine der Gruppen

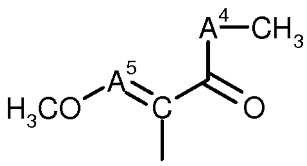

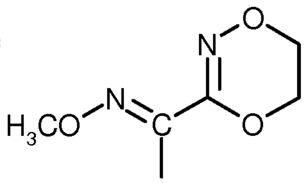

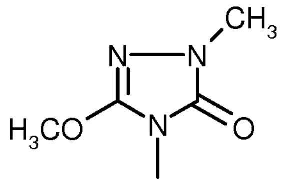

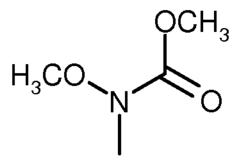

steht,

$A^4$ für NH oder O steht,

$A^5$ für N oder CH steht,

L für eine der Gruppen

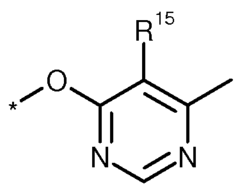

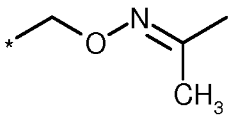

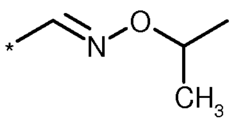

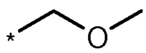

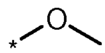

steht, wobei die Bindung, die mit einem Stern (*) markiert ist, an den Phenylring gebunden ist,

$R^{14}$ für jeweils gegebenenfalls einfach oder zweifach, gleich oder verschieden durch Chlor, Cyano, Methyl oder Trifluormethyl substituiertes Phenyl, Phenoxy oder Pyridinyl, oder für 1-(4-Chlorphenyl)-pyrazol-3-yl oder für 1,2-Propandion-bis(O-methyloxim)-1-yl steht,

$R^{15}$ für Wasserstoff oder Fluor steht;

Gruppe (3) Triazole der allgemeinen Formel (III)

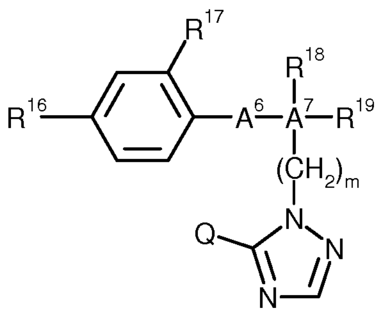

(III)

in welcher

Q für Wasserstoff oder SH steht,
m für 0 oder 1 steht,
$R^{16}$ für Wasserstoff, Fluor, Chlor, Phenyl oder 4-Chlor-phenoxy steht,
$R^{17}$ für Wasserstoff oder Chlor steht,
$A^6$ für eine direkte Bindung, $-CH_2-$, $-(CH_2)_2-$ oder -O- steht,
$A^6$ außerdem für $*-CH_2-CHR^{20}-$ oder $*-CH=CR^{20}-$ steht, wobei die mit * markierte Bindung mit dem Phenylring verknüpft ist, und
$R^{18}$ und $R^{20}$ dann zusammen für $-CH_2-CH_2-CH[CH(CH_3)_2]-$ oder $-CH_2-CH_2-C(CH_3)_2-$ stehen,
$A^7$ für C oder Si (Silizium) steht,
$A^6$ außerdem für $-N(R^{20})-$ steht und $A^7$ außerdem zusammen mit $R^{18}$ und $R^{19}$ für die Gruppe $C=N-R^{21}$ steht, wobei $R^{20}$ und $R^{21}$ dann zusammen für die Gruppe

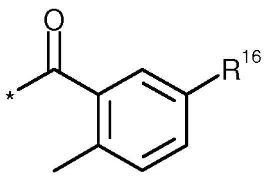

stehen, wobei die mit * markierte Bindung mit $R^{20}$ verbunden ist,
$R^{18}$ für Wasserstoff, Hydroxy oder Cyano steht,
$R^{19}$ für 1-Cyclopropylethyl, 1-Chlorcyclopropyl, $C_1$-$C_4$-Alkyl, $C_1$-$C_6$-Hydroxyalkyl, $C_1$-$C_4$-Alkylcarbonyl, $C_1$-$C_2$-Halogenalkoxy-$C_1$-$C_2$-alkyl, Trimethylsilyl-$C_1$-$C_2$-alkyl, Monofluorphenyl, oder Phenyl steht,
$R^{18}$ und $R^{19}$ außerdem zusammen für $-O-CH_2-CH(R^{21})-O-$, $-O-CH_2-CH(R^{21})-CH_2-$, oder -O-CH-(2-Chlorphenyl)- stehen,
$R^{20}$ zusammen mit $R^{18}$ für $-CH_2-CH_2-CH[CH(CH_3)_2]-$ oder $-CH_2-CH_2-C(CH_3)_2-$ steht, oder
$R^{20}$ zusammen mit $R^{21}$ für die Gruppe

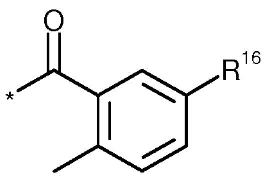

steht, wobei die mit * markierte Bindung mit $R^{20}$ verbunden ist,
$R^{21}$ für Wasserstoff, $C_1$-$C_4$-Alkyl oder Brom steht;

Gruppe (4) Sulfenamide der allgemeinen Formel (IV)

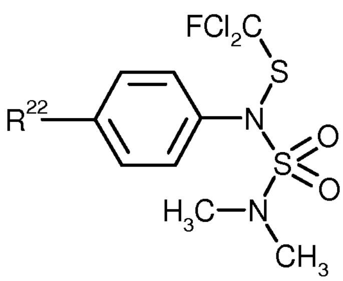

(IV)

in welcher $R^{22}$ für Wasserstoff oder Methyl steht;
Gruppe (5) Valinamide ausgewählt aus

(5-1) Iprovalicarb

(5-2) *N*[1]-[2-(4-{[3-(4-chlorophenyl)-2-propynyl]oxy}-3-methoxyphenyl)ethyl]-*N*[2]-(methylsulfonyl)-D-valinamid

(5-3) Benthiavalicarb

Gruppe (6) Carboxamide der allgemeinen Formel (V)

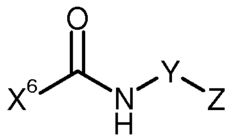

(V)

in welcher

$X^6$ für 2-Chlor-3-pyridinyl, für 1-Methylpyrazol-4-yl, welches in 3-Position durch Methyl oder Trifluormethyl und in 5-Position durch Wasserstoff oder Chlor substituiert ist, für 4-Ethyl-2-ethylamino-1,3-thiazol-5-yl, für 1-Methyl-cyclohexyl, für 2,2-Dichlor-1-ethyl-3-methyl-cyclopropyl, für 2-Fluor-2-propyl, oder für Phenyl steht, welches einfach bis dreifach, gleich oder verschieden durch Chlor oder Methyl substituiert ist, steht,

$X^6$ außerdem für 3,4-Dichlor-isothiazol-5-yl, 5,6-Dihydro-2-methyl-1,4-oxathiin-3-yl, 4-Methyl1,2,3-thiadiazol-5-yl, 4,5-Dimethyl-2-trimethylsilyl-thiophen-3-yl, 1-Methylpyrrol-3-yl, welches in 4-Position durch Methyl oder Trifluormethyl und in 5-Position durch Wasserstoff oder Chlor substituiert ist, steht,

Y für eine direkte Bindung, gegebenenfalls durch Chlor, Cyano oder Oxo substituiertes $C_1$-$C_6$-Alkandiyl (Alkylen) oder Thiophendiyl steht,

Y außerdem für $C_2$-$C_6$-Alkendiyl (Alkenylen) steht,

Z für Wasserstoff oder die Gruppe

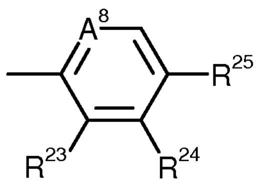

steht,

Z außerdem für $C_1$-$C_6$-Alkyl steht,

$A^8$ für CH oder N steht,

$R^{23}$ für Wasserstoff, Chlor, durch gegebenenfalls einfach oder zweifach, gleich oder verschieden durch Chlor oder Di($C_1$-$C_3$-alkyl)aminocarbonyl substituiertes Phenyl steht,

$R^{23}$ außerdem für Cyano oder $C_1$-$C_6$-Alkyl steht,

$R^{24}$ für Wasserstoff oder Chlor steht,

$R^{25}$ für Wasserstoff, Chlor, Hydroxy, Methyl oder Trifluormethyl steht,

$R^{25}$ außerdem für Di($C_1$-$C_3$-alkyl)aminocarbonyl steht,

$R^{23}$ und $R^{24}$ außerdem gemeinsam für *-$CH(CH_3)$-$CH_2$-$C(CH_3)_2$- oder *-$CH(CH_3)$-O-$C(CH_3)_2$- steht, wobei die mit * markierte Bindung mit $R^{23}$ verknüpft ist;

Gruppe (7) Dithiocarbamate ausgewählt aus

(7-1) Mancozeb
(7-2) Maneb

(7-3) Metiram
(7-4) Propineb
(7-5) Thiram
(7-6) Zineb
(7-7) Ziram

Gruppe (8) Acylalanine der allgemeinen Formel (VI)

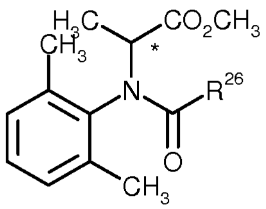

(VI)

in welcher

* ein Kohlenstoffatom in der R- oder der S-Konfiguration, bevorzugt in der S-Konfiguration, kennzeichnet,

$R^{26}$ für Benzyl, Furyl oder Methoxymethyl steht;

Gruppe (9): Anilino-pyrimidine der allgemeinen Formel (VII)

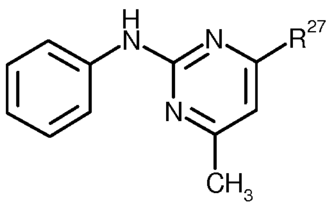

(VII)

in welcher

$R^{27}$ für Methyl, Cyclopropyl oder 1-Propinyl steht;

Gruppe (10): Benzimidazole der allgemeinen Formel (VIII)

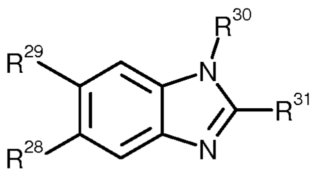

(VIII)

in welcher

$R^{28}$ und $R^{29}$ jeweils für Wasserstoff oder zusammen für $-O-CF_2-O-$ stehen,

$R^{30}$ für Wasserstoff, $C_1$-$C_4$-Alkylaminocarbonyl oder für 3,5-Dimethylisoxazol-4-ylsulfonyl steht,

$R^{31}$ für Chlor, Methoxycarbonylamino, Chlorphenyl, Furyl oder Thiazolyl steht;

Gruppe (11): Carbamate der allgemeinen Formel (IX)

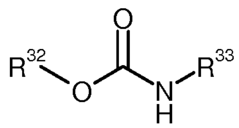

(IX)

in welcher

$R^{32}$ für n- oder iso-Propyl steht,
$R^{33}$ für Di($C_1$-$C_2$-alkyl)amino-$C_2$-$C_4$-alkyl oder Diethoxyphenyl steht,

wobei auch Salz dieser Verbindungen eingeschlossen sind;
Gruppe (12): Dicarboximide ausgewählt aus

(12-1) Captafol
(12-2) Captan
(12-3) Folpet
(12-4) Iprodione
(12-5) Procymidone
(12-6) Vinclozolin

Gruppe (13): Guanidine ausgewählt aus

(13-1) Dodine
(13-2) Guazatine
(13-3) Iminoctadine triacetate
(13-4) Iminoctadine tris(albesilate)

Gruppe (14): Imidazole ausgewählt aus

(14-1) Cyazofamid
(14-2) Prochloraz
(14-3) Triazoxide
(14-4) Pefurazoate

Gruppe (15): Morpholine der allgemeinen Formel (X)

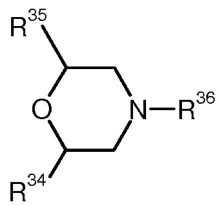

(X)

in welcher

$R^{34}$ und $R^{35}$ unabhängig voneinander für Wasserstoff oder Methyl stehen,

$R^{36}$ für $C_1$-$C_{14}$-Alkyl (bevorzugt $C_{12}$-$C_{14}$-Alkyl), $C_5$-$C_{12}$-Cycloalkyl (bevorzugt $C_{10}$-$C_{12}$-Cycloalkyl), Phenyl-$C_1$-$C_4$-alkyl, welches im Phenylteil durch Halogen oder $C_1$-$C_4$-Alkyl substituiert sein kann, oder für Acrylyl, welches durch Chlorphenyl und Dimethoxyphenyl substituiert ist, steht;

Gruppe (16): Pyrrole der allgemeinen Formel (XI)

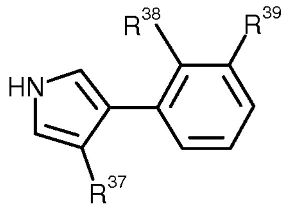

(XI)

in welcher

$R^{37}$ für Chlor oder Cyano steht,

$R^{38}$ für Chlor oder Nitro steht,

$R^{39}$ für Chlor steht

$R^{38}$ und $R^{39}$ außerdem gemeinsam für -O-$CF_2$-O- stehen;

Gruppe (17): Phosphonate ausgewählt aus

(17-1) Fosetyl-Al
(17-2) Phosphonsäure;

Gruppe (18): Phenylethanamide der allgemeinen Formel (XII)

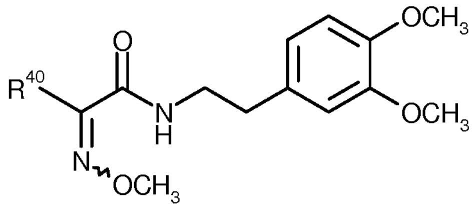

(XII)

in welcher

$R^{40}$ für unsubstituiertes oder durch Fluor, Chlor, Brom, Methyl oder Ethyl substituiertes Phenyl, 2-Naphthyl, 1,2,3,4-Tetrahydronaphthyl oder Indanyl steht;

Gruppe (19): Fungizide ausgewählt aus

(19-1) Acibenzolar-S-methyl
(19-2) Chlorothalonil
(19-3) Cymoxanil
(19-4) Edifenphos
(19-5) Famoxadone
(19-6) Fluazinam
(19-7) Kupferoxychlorid
(19-8) Kupferhydroxid
(19-9) Oxadixyl
(19-10) Spiroxamine
(19-11) Dithianon
(19-12) Metrafenone
(19-13) Fenamidone
(19-14) 2,3-Dibutyl-6-chlor-thieno[2,3-d]pyrimidin-4(3H)on
(19-15) Probenazole
(19-16) Isoprothiolane
(19-17) Kasugamycin
(19-18) Phthalide

(19-19) Ferimzone
(19-20) Tricyclazole
(19-21) N-({4-[(Cyclopropylamino)carbonyl]phenyl}sulfonyl)-2-methoxybenzamid

Gruppe (20): (Thio)Harnstoff-Derivate ausgewählt aus

(20-1) Pencycuron
(20-2) Thiophanate-methyl
(20-3) Thiohanate-ethyl

Gruppe (21): Amide der allgemeinen Formel (XIII)

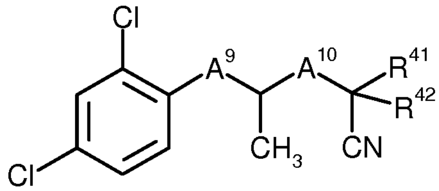

(XIII)

in welcher

$A^9$ für eine direkte Bindung oder -O- steht,

$A^{10}$ für -C(=O)NH- oder -NHC(=O)- steht,

$R^{41}$ für Wasserstoff oder $C_1$-$C_4$-Alkyl steht,

$R^{42}$ für $C_1$-$C_6$-Alkyl steht;

Gruppe (22): Triazolopyrimidine der allgemeinen Formel (XIV)

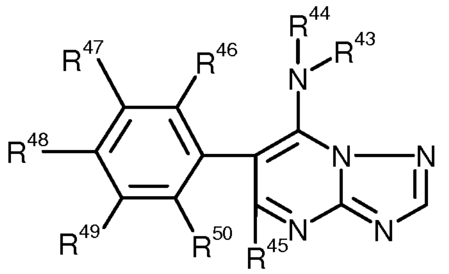

(XIV)

in welcher

$R^{43}$ für $C_1$-$C_6$-Alkyl oder $C_2$-$C_6$-Alkenyl steht,

$R^{44}$ für $C_1$-$C_6$-Alkyl steht,

$R^{43}$ und $R^{44}$ außerdem gemeinsam für $C_4$-$C_5$-Alkandiyl (Alkylen) stehen, welches einfach oder zweifach durch $C_1$-$C_6$-Alkyl substituiert ist,

$R^{45}$ für Brom oder Chlor steht,

$R^{46}$ und $R^{50}$ unabhängig voneinander für Wasserstoff, Fluor, Chlor oder Methyl stehen,

$R^{47}$ und $R^{49}$ unabhängig voneinander für Wasserstoff oder Fluor stehen,

$R^{48}$ für Wasserstoff, Fluor oder Methyl steht,

Gruppe (23): Iodochromone der allgemeinen Formel (XV)

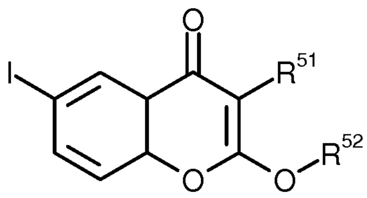

(XV)

in welcher

$R^{51}$ für $C_1$-$C_6$-Alkyl steht,

$R^{52}$ für $C_1$-$C_6$-Alkyl, $C_2$-$C_6$-Alkenyl oder $C_2$-$C_6$-Alkinyl steht;

Gruppe (24): Biphenylcarboxamide der allgemeinen Formel (XVI)

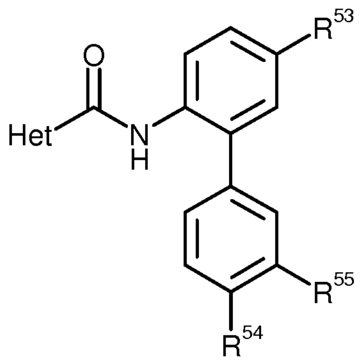

(XVI)

in welcher

$R^{53}$ für Wasserstoff oder Fluor steht,

$R^{54}$ für Fluor, Chlor, Brom, Methyl, Trifluormethyl, Trifluormethoxy, -CH=N-OMe oder -C(Me)=N-OMe steht,

$R^{55}$ für Wasserstoff, Fluor, Chlor, Brom, Methyl oder Trifluormethyl steht,

Het für einen der folgenden Reste Het1 bis Het7 steht:

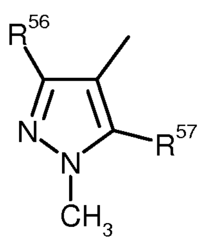

Het1

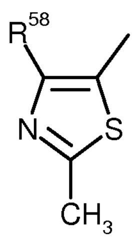

Het2

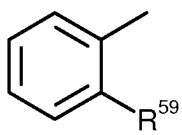

Het3

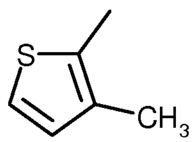

Het4

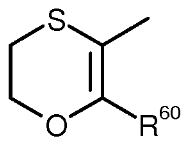

Het5

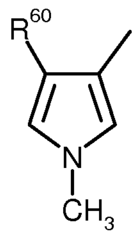

Het6

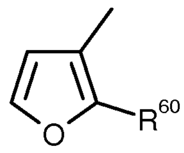

Het7

$R^{56}$ für Iod, Methyl, Difluormethyl oder Trifluormethyl steht,

$R^{57}$ für Wasserstoff, Fluor, Chlor oder Methyl steht,

$R^{58}$ für Methyl, Difluormethyl oder Trifluormethyl steht,

$R^{59}$ für Chlor, Brom, Iod, Methyl, Difluormethyl oder Trifluormethyl steht,

$R^{60}$ für Methyl oder Trifluormethyl steht.

Die Formel (II) umfasst folgende bevorzugte Kombinationspartner der Gruppe (2):

(2-1) Azoxystrobin (bekannt aus EP-A 0 382 375) der Formel

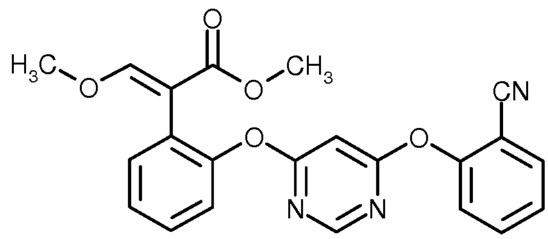

(2-2) Fluoxastrobin (bekannt aus DE-A 196 02 095) der Formel

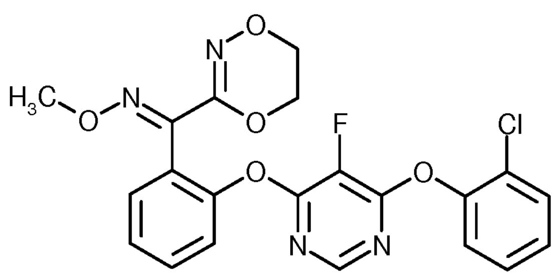

(2-3) (2*E*)-2-(2-{[6-(3-Chlor-2-methylphenoxy)-5-fluor-4-pyrimidinyl]oxy}phenyl)-2-(methoxyimino)-*N*-methylethanamid (bekannt aus DE-A 196 46 407, EP-B 0 712 396) der Formel

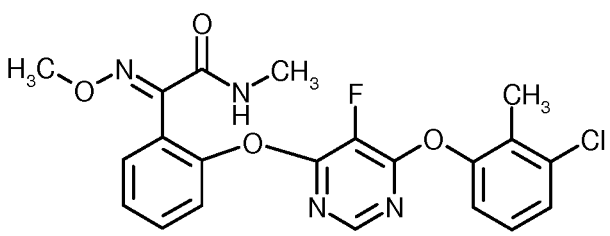

(2-4) Trifloxystrobin (bekannt aus EP-A 0 460 575) der Formel

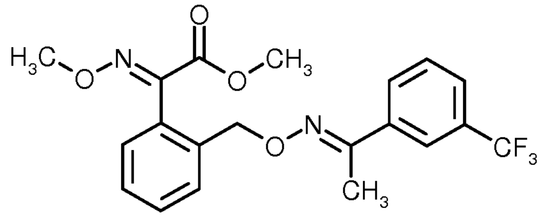

(2-5) (2*E*)-2-(Methoxyimino)-*N*-methyl-2-(2-{[({(1*E*)-1-[3-(trifluormethyl)phenyl]ethyliden}-amino)oxy]methyl}phenyl)ethanamid (bekannt aus EP-A 0 569 384) der Formel

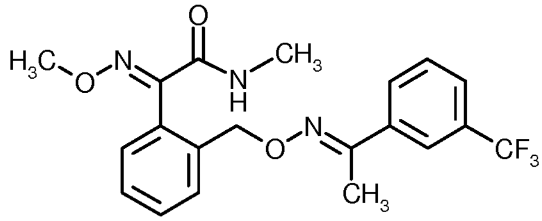

(2-6) (2*E*)-2-(Methoxyimino)-*N*-methyl-2-{2-[(*E*)-({1-[3-(trifluormethyl)phenyl]ethoxy}imino)-methyl]phenyl}ethanamid (bekannt aus EP-A 0 596 254) der Formel

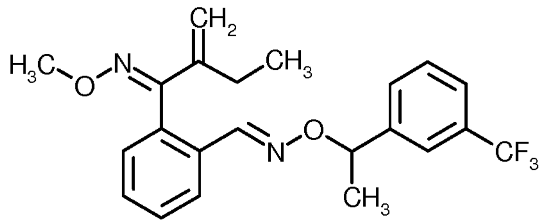

(2-7) Orysastrobin (bekannt aus DE-A 195 39 324) der Formel

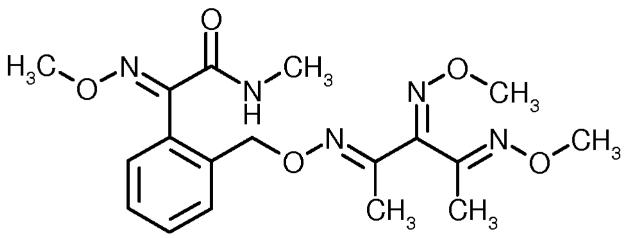

(2-8) 5-Methoxy-2-methyl-4-(2-{[({(1*E*)-1-[3-(trifluormethyl)phenyl]ethyliden}amino)oxy]-methyl}phenyl)-2,4-dihydro-3*H*-1,2,4-triazol-3-on (bekannt aus WO 98/23155) der Formel

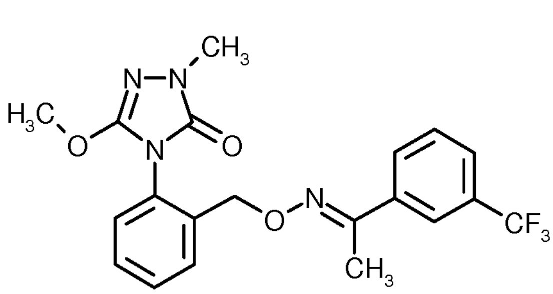

(2-9) Kresoxim-methyl (bekannt aus EP-A 0 253 213) der Formel

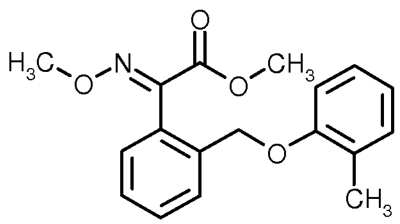

(2-10) Dimoxystrobin (bekannt aus EP-A 0 398 692) der Formel

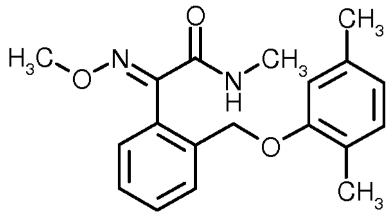

(2-11) Picoxystrobin (bekannt aus EP-A 0 278 595) der Formel

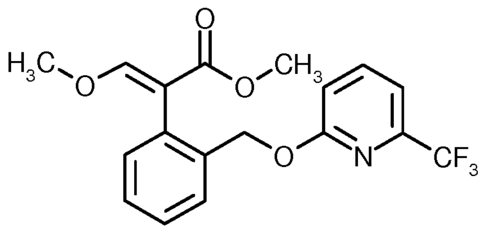

(2-12) Pyraclostrobin (bekannt aus DE-A 44 23 612) der Formel

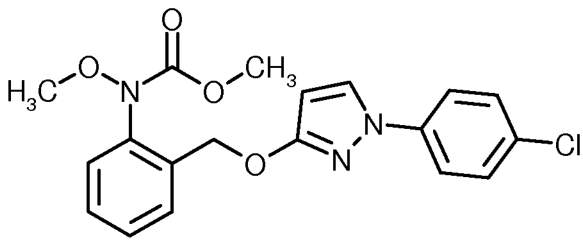

(2-13) Metominostrobin (bekannt aus EP-A 0 398 692) der Formel

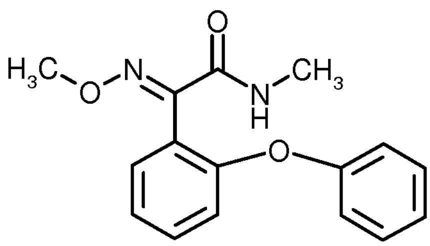

Die Formel (III) umfasst folgende bevorzugte Kombinationspartner der Gruppe (3):

(3-1) Azaconazole (bekannt aus DE-A 25 51 560) der Formel

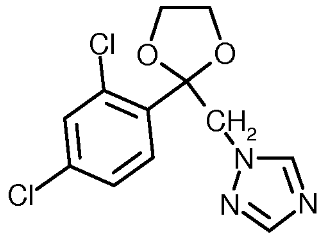

(3-2) Etaconazole (bekannt aus DE-A 25 51 560) der Formel

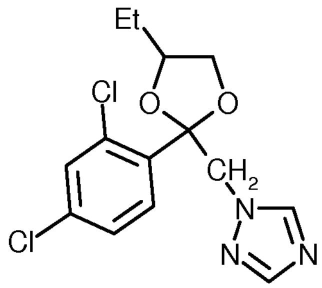

(3-3) Propiconazole (bekannt aus DE-A 25 51 560) der Formel

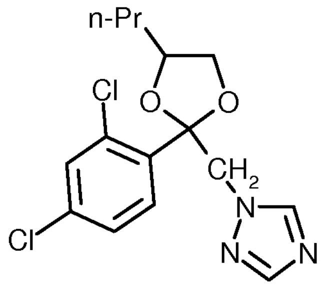

(3-4) Difenoconazole (bekannt aus EP-A 0 112 284) der Formel

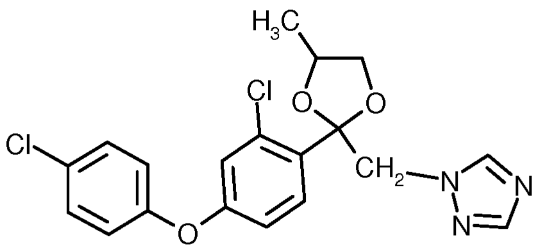

(3-5) Bromuconazole (bekannt aus EP-A 0 258 161) der Formel

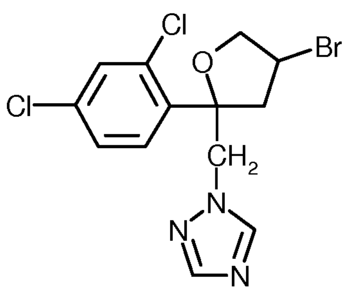

(3-6) Cyproconazole (bekannt aus DE-A 34 06 993) der Formel

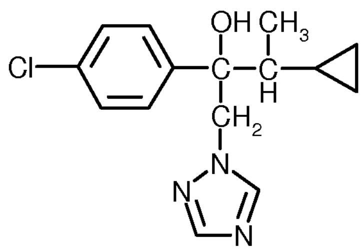

(3-7) Hexaconazole (bekannt aus DE-A 30 42 303) der Formel

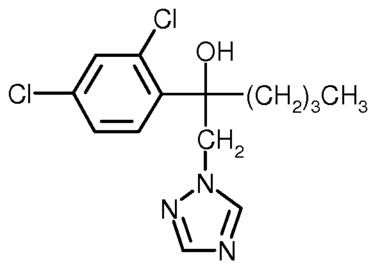

(3-8) Penconazole (bekannt aus DE-A 27 35 872) der Formel

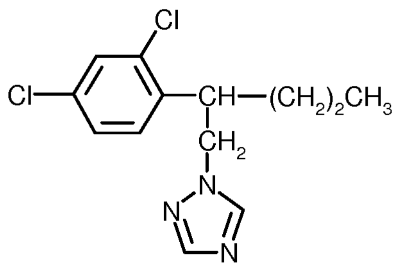

(3-9) Myclobutanil (bekannt aus EP-A 0 145 294) der Formel

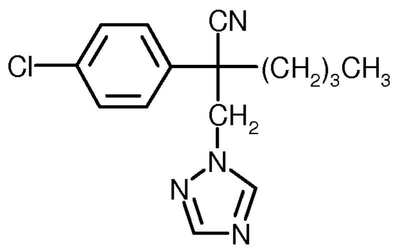

(3-10) Tetraconazole (bekannt aus EP-A 0 234 242) der Formel

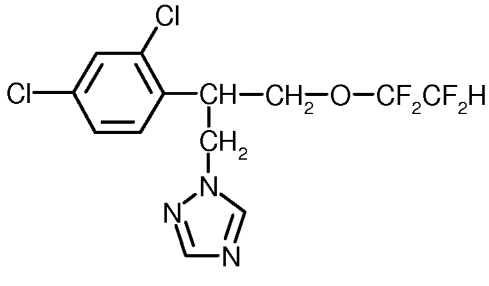

(3-11) Flutriafol (bekannt aus EP-A 0 015 756) der Formel

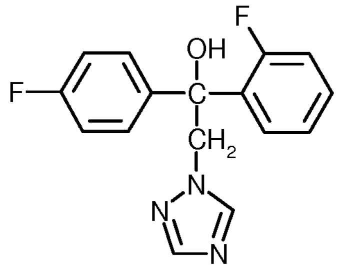

(3-12) Epoxiconazole (bekannt aus EP-A 0 196 038) der Formel

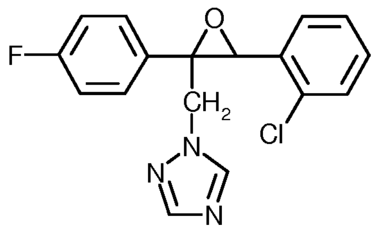

(3-13) Flusilazole (bekannt aus EP-A 0 068 813) der Formel

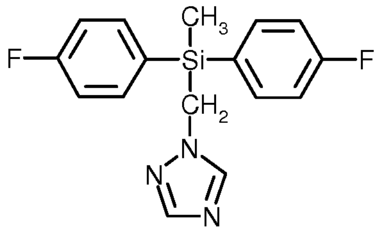

(3-14) Simeconazole (bekannt aus EP-A 0 537 957) der Formel

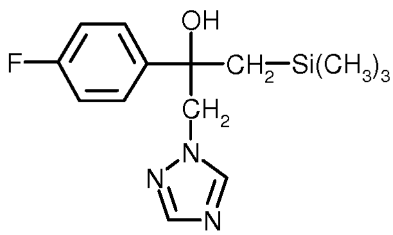

(3-15) Prothioconazole (bekannt aus WO 96/16048) der Formel

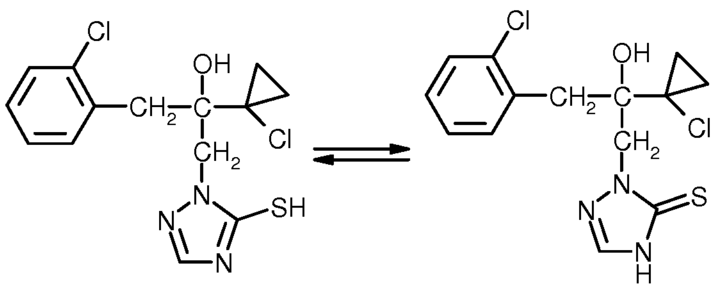

(3-16) Fenbuconazole (bekannt aus DE-A 37 21 786) der Formel

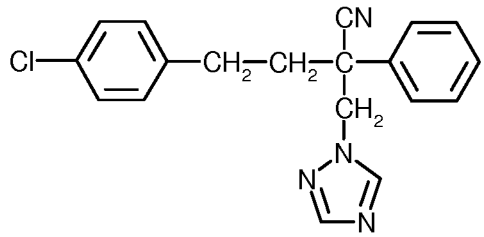

(3-17) Tebuconazole (bekannt aus EP-A 0 040 345) der Formel

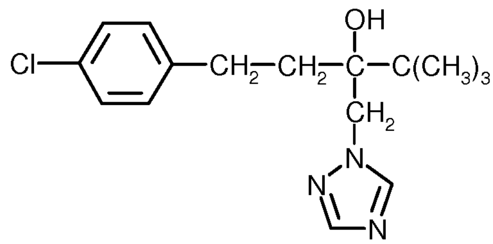

(3-18) Ipconazole (bekannt aus EP-A 0 329 397) der Formel

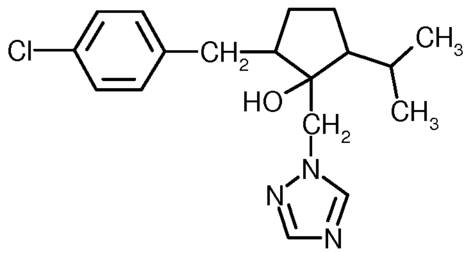

(3-19) Metconazole (bekannt aus EP-A 0 329 397) der Formel

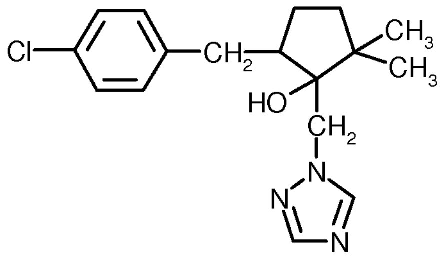

(3-20) Triticonazole (bekannt aus EP-A 0 378 953) der Formel

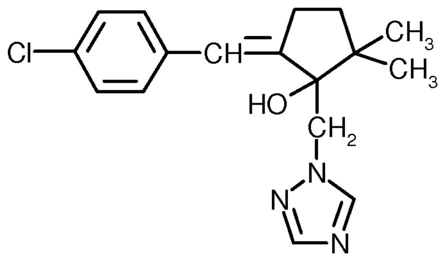

(3-21) Bitertanol (bekannt aus DE-A 23 24 010) der Formel

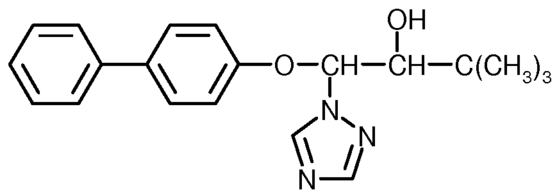

(3-22) Triadimenol (bekannt aus DE-A 23 24 010) der Formel

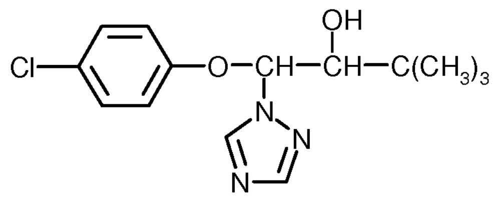

(3-23) Triadimefon (bekannt aus DE-A 22 01 063) der Formel

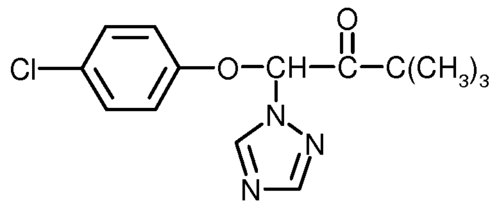

(3-24) Fluquinconazole (bekannt aus EP-A 0 183 458) der Formel

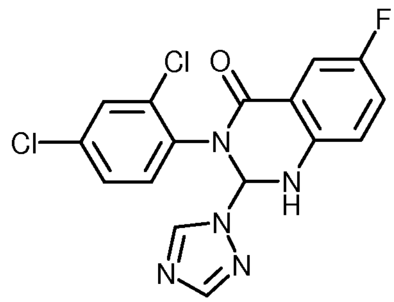

(3-25) Quinconazole (bekannt aus EP-A 0 183 458) der Formel

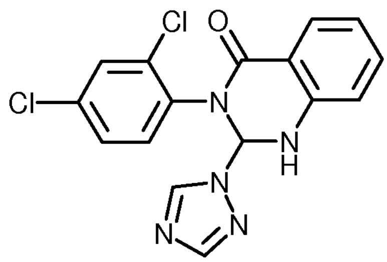

Die Formel (IV) umfasst folgende bevorzugte Kombinationspartner der Gruppe (4):

(4-1) Dichlofluanid (bekannt aus DE-A 11 93 498) der Formel

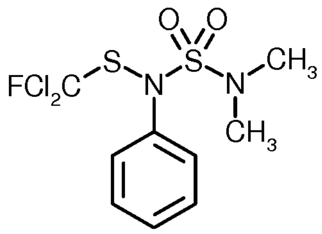

(4-2) Tolylfluanid (bekannt aus DE-A 11 93 498) der Formel

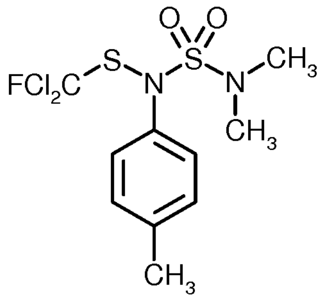

Bevorzugte Kombinationspartner der Gruppe (5) sind

(5-1) Iprovalicarb (bekannt aus DE-A 40 26 966) der Formel

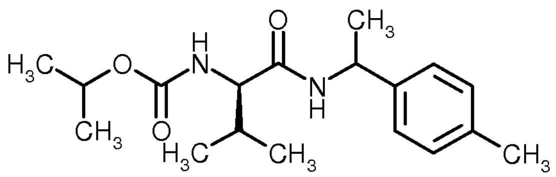

(5-3) Benthiavalicarb (bekannt aus WO 96/04252) der Formel

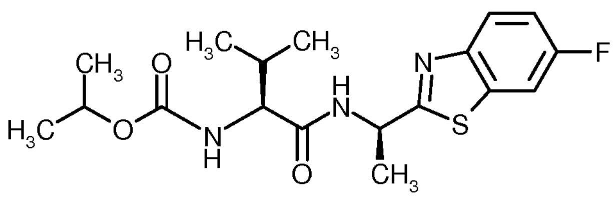

Die Formel (V) umfasst folgende bevorzugte Kombinationspartner der Gruppe (6):

(6-1) 2-Chloro-N-(1,1,3-trimethyl-indan-4-yl)-nicotinamid (bekannt aus EP-A 0 256 503) der Formel

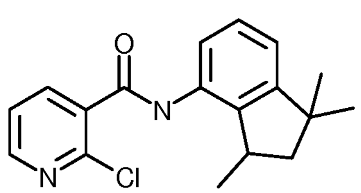

(6-2) Boscalid (bekannt aus DE-A 195 31 813) der Formel

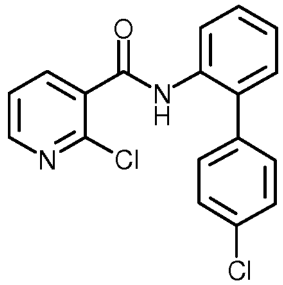

(6-3) Furametpyr (bekannt aus EP-A 0 315 502) der Formel

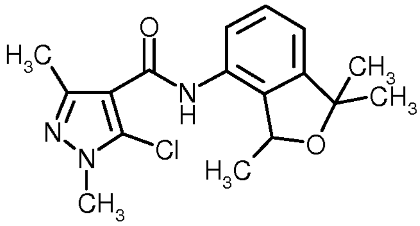

(6-4) 1-Methyl-3-trifluormethyl-1H-pyrazol-4-carbonsäure-(3-p-tolyl-thiophen-2-yl)-amid (bekannt aus EP-A 0 737 682) der Formel

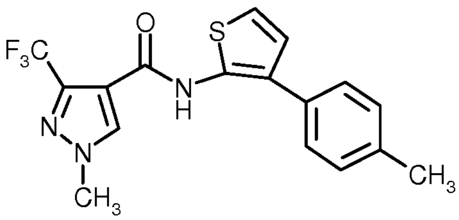

(6-5) Ethaboxam (bekannt aus EP-A 0 639 574) der Formel

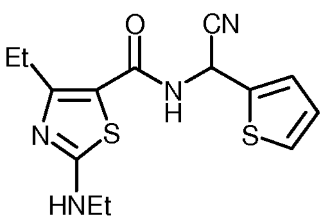

(6-6) Fenhexamid (bekannt aus EP-A 0 339 418) der Formel

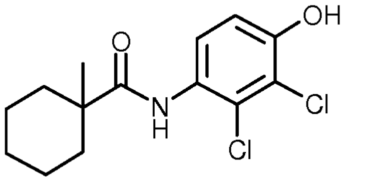

(6-7) Carpropamid (bekannt aus EP-A 0 341 475) der Formel

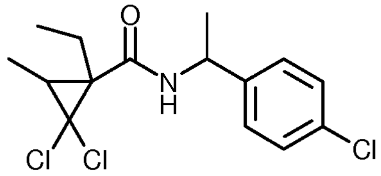

(6-8) 2-Chlor-4-(2-fluor-2-methyl-propionylamino)-N,N-dimethyl-benzamid (bekannt aus EP-A 0 600 629) der Formel

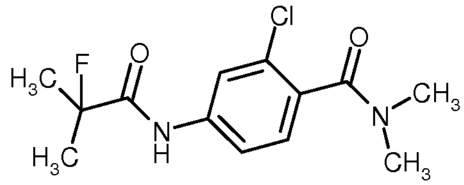

(6-9) Picobenzamid (bekannt aus WO 99/42447) der Formel

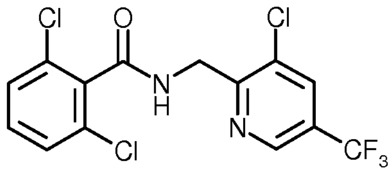

(6-10) Zoxamide (bekannt aus EP-A 0 604 019) der Formel

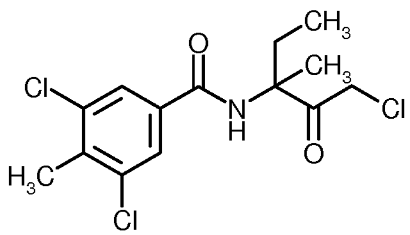

(6-11) 3,4-Dichlor-N-(2-cyanophenyl)isothiazol-5-carboxamid (bekannt aus WO 99/24413) der Formel

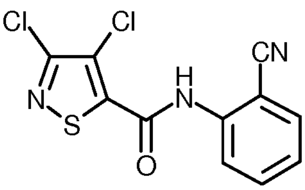

(6-12) Carboxin (bekannt aus US 3,249,499) der Formel

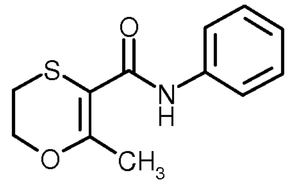

(6-13) Tiadinil (bekannt aus US 6,616,054) der Formel

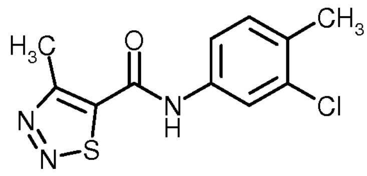

(6-14) Penthiopyrad (bekannt aus EP-A 0 737 682) der Formel

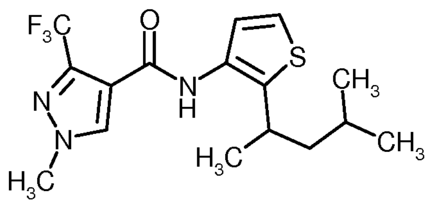

(6-15) Silthiofam (bekannt aus WO 96/18631) der Formel

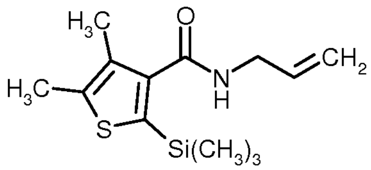

(6-16) *N*-[2-(1,3-Dimethylbutyl)phenyl]-1-methyl-4-(trifluormethyl)-1*H*-pyrrol-3-carboxamid (bekannt aus WO 02/38542) der Formel

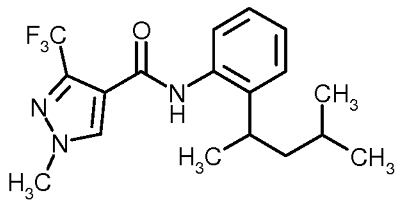

Bevorzugte Kombinationspartner der Gruppe (7) sind

(7-1) Mancozeb (bekannt aus DE-A 12 34 704) mit dem IUPAC-Namen
Mangan-Ethylenbis(dithiocarbamat) (polymer) Komplex mit Zinksalz
(7-2) Maneb (bekannt aus US 2,504,404) der Formel

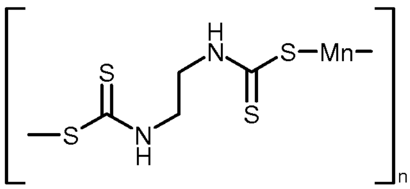

(7-3) Metiram (bekannt aus DE-A 10 76 434) mit dem IUPAC-Namen
Zink Ammoniak Ethylenbis(dithiocarbamat) - poly(ethylenthiuram disulfid)
(7-4) Propineb (bekannt aus GB 935 981) der Formel

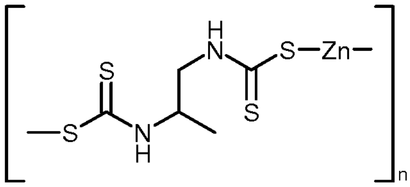

(7-5) Thiram (bekannt aus US 1,972,961) der Formel

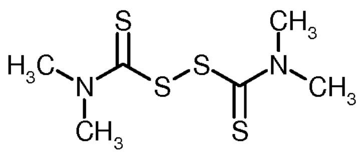

(7-6) Zineb (bekannt aus DE-A 10 81 446) der Formel

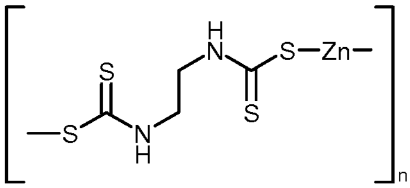

(7-7) Ziram (bekannt aus US 2,588,428) der Formel

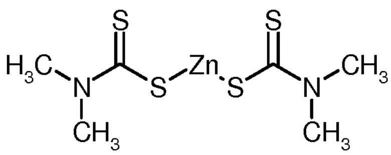

Die Formel (VI) umfasst folgende bevorzugte Kombinationspartner der Gruppe (8):

(8-1) Benalaxyl (bekannt aus DE-A 29 03 612) der Formel

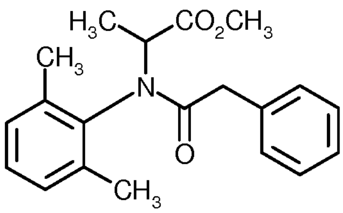

(8-2) Furalaxyl (bekannt aus DE-A 25 13 732) der Formel

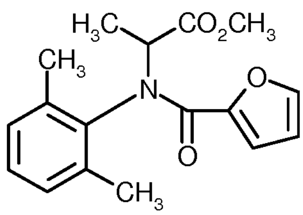

(8-3) Metalaxyl (bekannt aus DE-A 25 15 091) der Formel

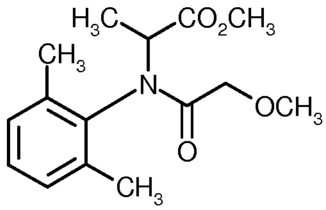

(8-4) Metalaxyl-M (bekannt aus WO 96/01559) der Formel

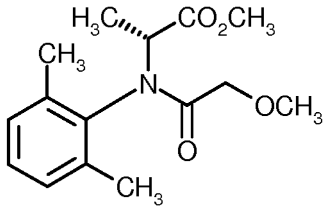

(8-5) Benalaxyl-M der Formel

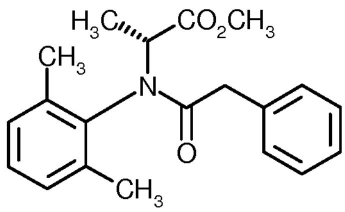

Die Formel (VII) umfasst folgende bevorzugte Kombinationspartner der Gruppe (9):

(9-1) Cyprodinil (bekannt aus EP-A 0 310 550) der Formel

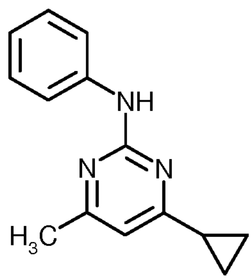

(9-2) Mepanipyrim (bekannt aus EP-A 0 270 111) der Formel

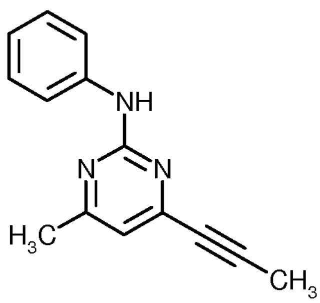

(9-3) Pyrimethanil (bekannt aus DD 151 404) der Formel

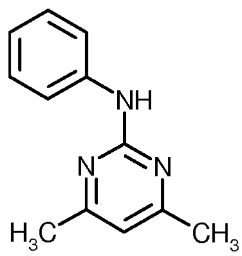

Die Formel (VIII) umfasst folgende bevorzugte Kombinationspartner der Gruppe (10):

(10-1) 6-Chlor-5-[(3,5-dimethylisoxazol-4-yl)sulfonyl]-2,2-difluor-5H-[1,3]dioxolo[4,5-f]-benzimidazol (bekannt aus WO 97/06171) der Formel

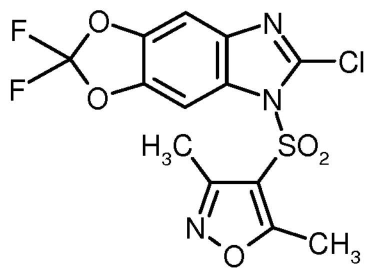

(10-2) Benomyl (bekannt aus US 3,631,176) der Formel

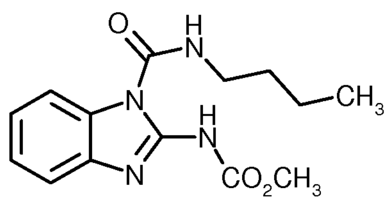

(10-3) Carbendazim (bekannt aus US 3,010,968) der Formel

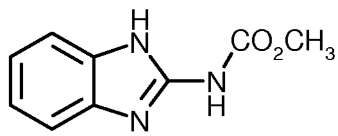

(10-4) Chlorfenazole der Formel

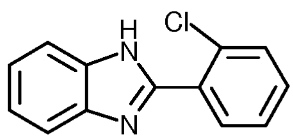

(10-5) Fuberidazole (bekannt aus DE-A 12 09 799) der Formel

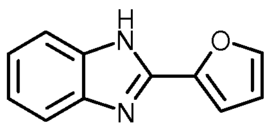

(10-6) Thiabendazole (bekannt aus US 3,206,468) der Formel

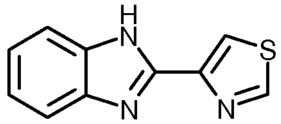

Die Formel (IX) umfasst folgende bevorzugte Kombinationspartner der Gruppe (11):

(11-1) Diethofencarb (bekannt aus EP-A 0 078 663) der Formel

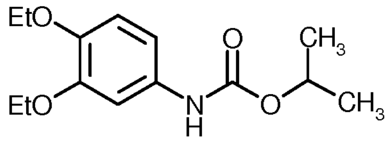

(11-2) Propamocarb (bekannt aus US 3,513,241) der Formel

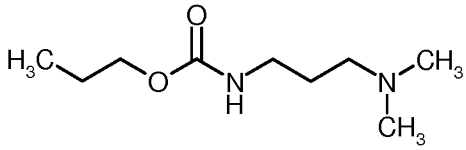

(11-3) Propamocarb-hydrochloride (bekannt aus US 3,513,241) der Formel

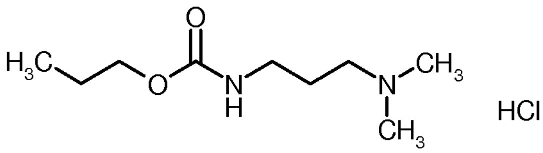

(11-4) Propamocarb-Fosetyl der Formel

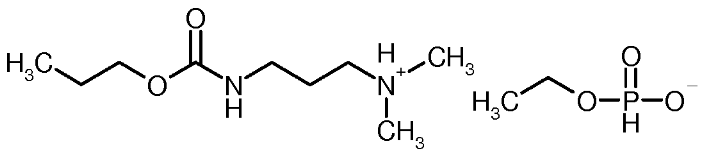

Bevorzugte Kombinationspartner der Gruppe (12) sind

(12-1) Captafol (bekannt aus US 3,178,447) der Formel

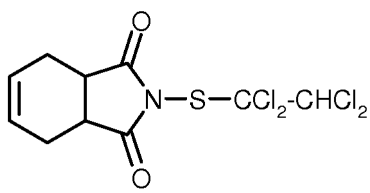

(12-2) Captan (bekannt aus US 2,553,770) der Formel

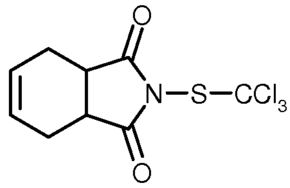

(12-3) Folpet (bekannt aus US 2,553,770) der Formel

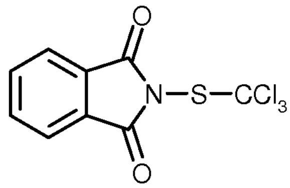

(12-4) Iprodione (bekannt aus DE-A 21 49 923) der Formel

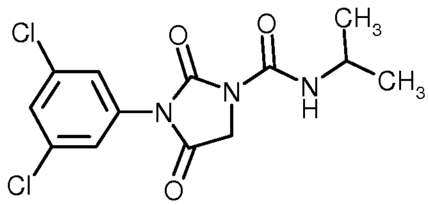

(12-5) Procymidone (bekannt aus DE-A 20 12 656) der Formel

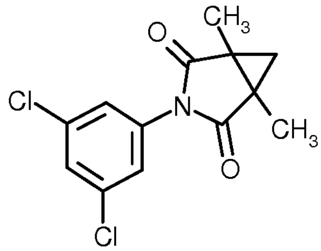

(12-6) Vinclozolin (bekannt aus DE-A 22 07 576) der Formel

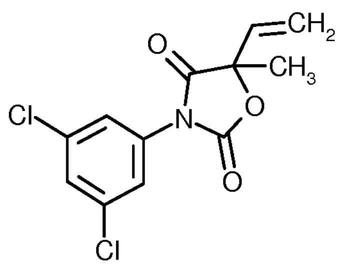

Bevorzugte Kombinationspartner der Gruppe (13) sind

(13-1) Dodine (bekannt aus GB 11 03 989) der Formel

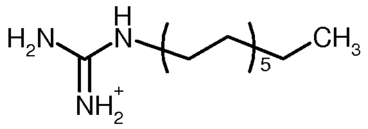

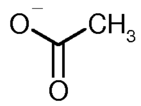

(13-2) Guazatine (bekannt aus GB 11 14 155)

(13-3) Iminoctadine triacetate (bekannt aus EP-A 0 155 509) der Formel

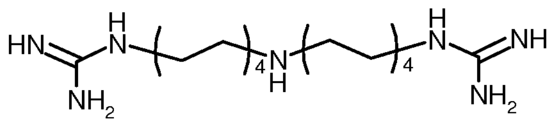

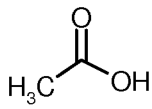

Bevorzugter Kombinationspartner der Gruppe (14) ist

(14-1) Cyazofamid (bekannt aus EP-A 0 298 196) der Formel

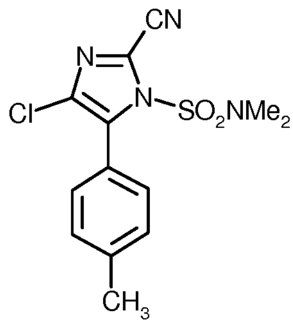

(14-2) Prochloraz (bekannt aus DE-A 24 29 523) der Formel

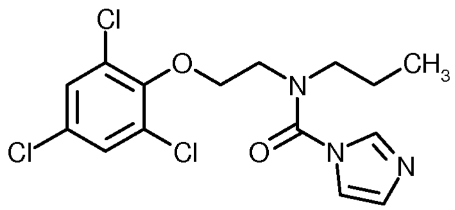

(14-3) Triazoxide (bekannt aus DE-A 28 02 488) der Formel

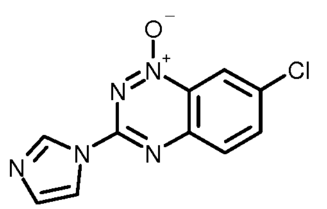

(14-4) Pefurazoate (bekannt aus EP-A 0 248 086) der Formel

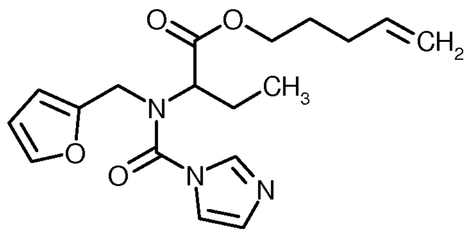

Die Formel (X) umfasst folgende bevorzugte Kombinationspartner der Gruppe (15):

(15-1) Aldimorph (bekannt aus DD 140 041) der Formel

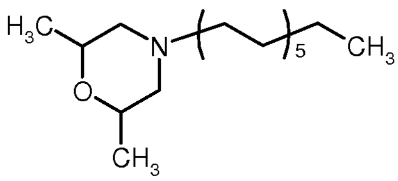

(15-2) Tridemorph (bekannt aus GB 988 630) der Formel

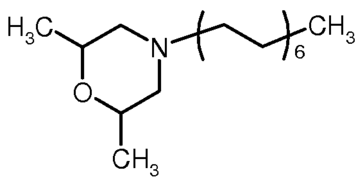

(15-3) Dodemorph (bekannt aus DE-A 25 432 79) der Formel

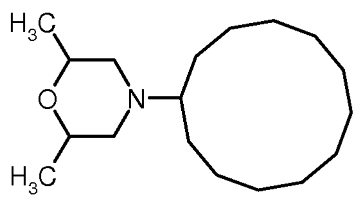

(15-4) Fenpropimorph (bekannt aus DE-A 26 56 747) der Formel

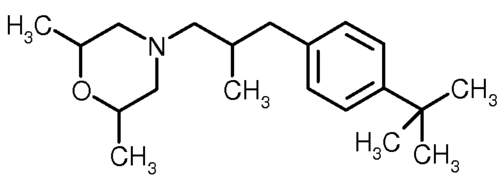

(15-5) Dimethomorph (bekannt aus EP-A 0 219 756) der Formel

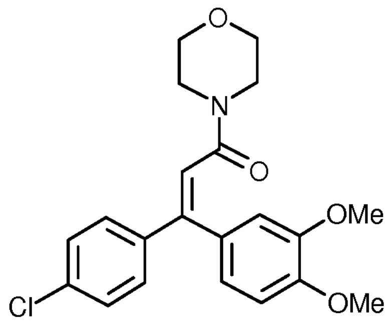

Die Formel (XI) umfasst folgende bevorzugte Kombinationspartner der Gruppe (16):

(16-1) Fenpiclonil (bekannt aus EP-A 0 236 272) der Formel

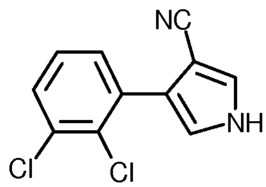

(16-2) Fludioxonil (bekannt aus EP-A 0 206 999) der Formel

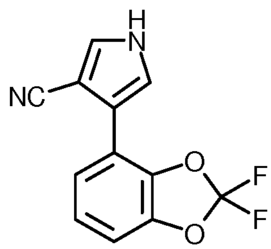

(16-3) Pyrrolnitrine (bekannt aus JP 65-25876) der Formel

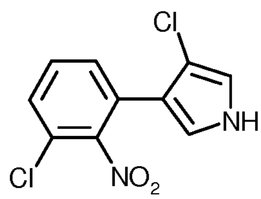

Bevorzugte Kombinationspartner der Gruppe (17) sind

(17-1) Fosetyl-Al (bekannt aus DE-A 24 56 627) der Formel

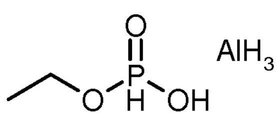

(17-2) Phosphonsäure (bekannte Chemikalie) der Formel

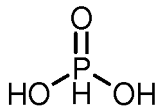

Die Formel (XII) umfasst folgende bevorzugte Kombinationspartner der Gruppe (18), welche aus WO 96/23793 bekannt sind und jeweils als E- oder Z-Isomere vorliegen können. Verbindungen der Formel (XII) können daher als Gemisch von verschiedenen Isomeren oder auch in Form eines einzigen Isomeren vorliegen. Bevorzugt sind Verbindungen der Formel (XII) in Form ihres E-Isomers:

(18-1) die Verbindung 2-(2,3-Dihydro-1H-inden-5-yl)-N-[2-(3,4-dimethoxyphenyl)ethyl]-2-(methoxyimino)acetamid der Formel

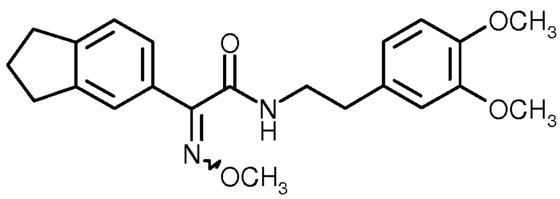

(18-2) die Verbindung N-[2-(3,4-Dimethoxyphenyl)ethyl]-2-(methoxyimino)-2-(5,6,7,8-tetrahydronaphthalen-2-yl)acetamid der Formel

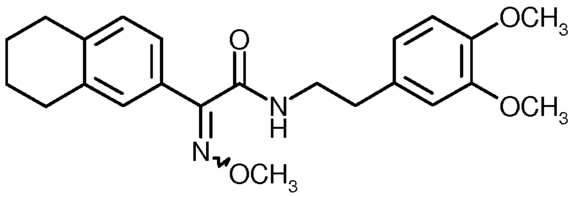

(18-3) die Verbindung 2-(4-Chlorphenyl)-N-[2-(3,4-dimethoxyphenyl)ethyl]-2-(methoxyimino)acetamid der Formel

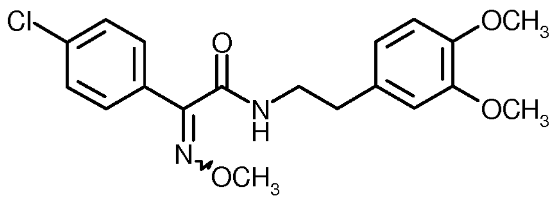

(18-4) die Verbindung 2-(4-Bromphenyl)-N-[2-(3,4-dimethoxyphenyl)ethyl]-2-(methoxyimino)acetamid der Formel

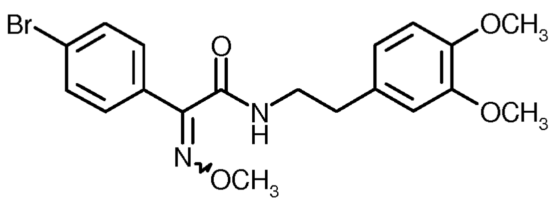

(18-5) die Verbindung 2-(4-Methylphenyl)-N-[2-(3,4-dimethoxyphenyl)ethyl]-2-(methoxyimino)-acetamid der Formel

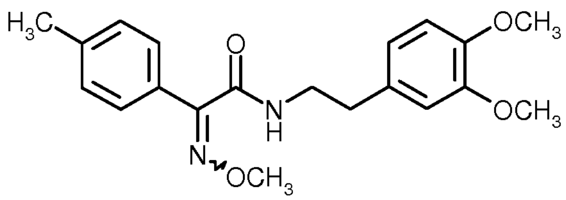

(18-6) die Verbindung 2-(4-Ethylphenyl)-N-[2-(3,4-dimethoxyphenyl)ethyl]-2-(methoxyimino)acetamid der Formel

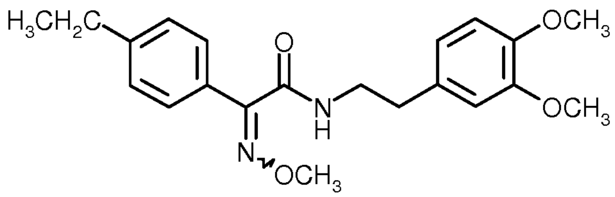

Bevorzugte Kombinationspartner der Gruppe (19) sind

(19-1) Acibenzolar-S-methyl (bekannt aus EP-A 0 313 512) der Formel

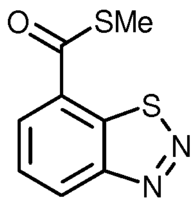

(19-2) Chlorothalonil (bekannt aus US 3,290,353) der Formel

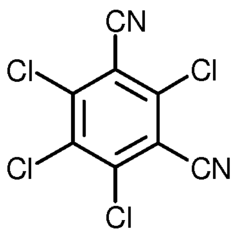

(19-3) Cymoxanil (bekannt aus DE-A 23 12 956) der Formel

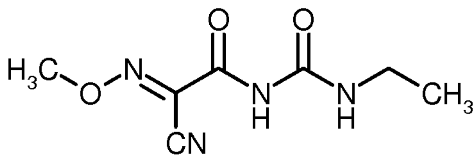

(19-4) Edifenphos (bekannt aus DE-A 14 93 736) der Formel

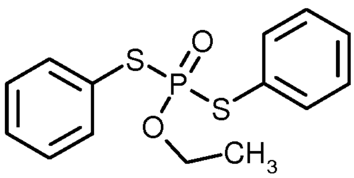

(19-5) Famoxadone (bekannt aus EP-A 0 393 911) der Formel

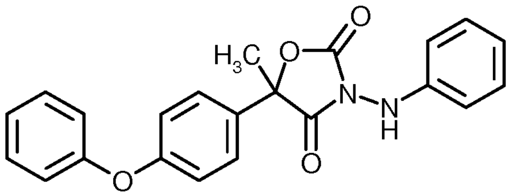

(19-6) Fluazinam (bekannt aus EP-A 0 031 257) der Formel

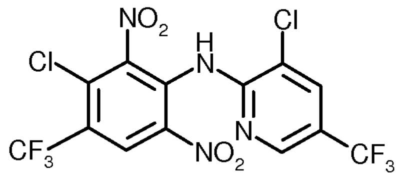

(19-7) Kupferoxychlorid

(19-9) Oxadixyl (bekannt aus DE-A 30 30 026) der Formel

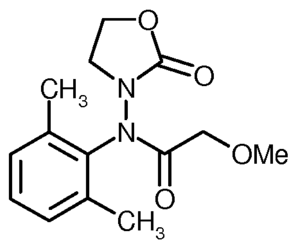

(19-10) Spiroxamine (bekannt aus DE-A 37 35 555) der Formel

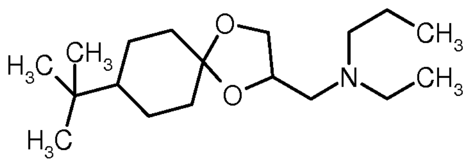

(19-11) Dithianon (bekannt aus JP-A 44-29464) der Formel

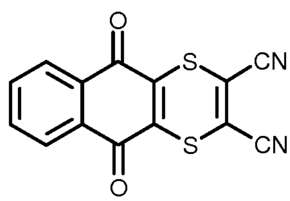

(19-12) Metrafenone (bekannt aus EP-A 0 897 904) der Formel

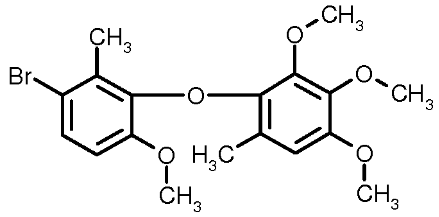

(19-13) Fenamidone (bekannt aus EP-A 0 629 616) der Formel

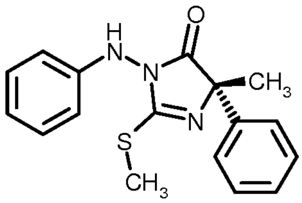

(19-14) 2,3-Dibutyl-6-chlor-thieno[2,3-d]pyrimidin-4(3H)on (bekannt aus WO 99/14202) der Formel

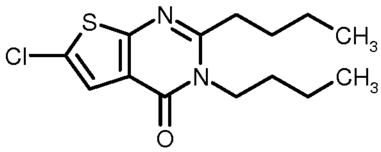

(19-15) Probenazole (bekannt aus US 3,629,428) der Formel

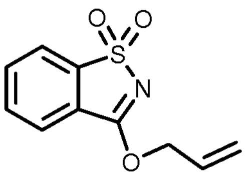

(19-16) Isoprothiolane (bekannt aus US 3,856,814) der Formel

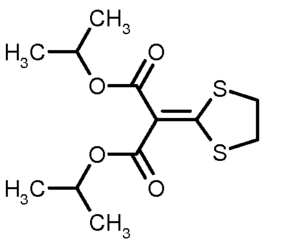

(19-17) Kasugamycin (bekannt aus GB 1 094 567) der Formel

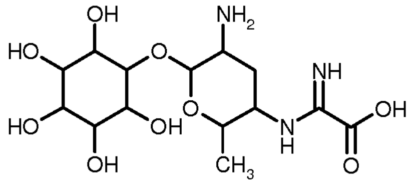

(19-18) Phthalide (bekannt aus JP-A 57-55844) der Formel

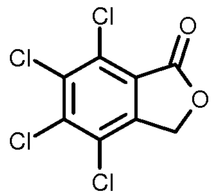

(19-19) Ferimzone (bekannt aus EP-A 0 019 450) der Formel

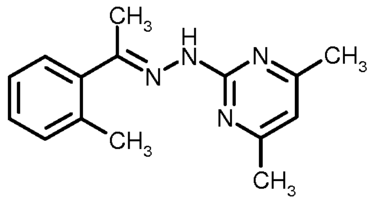

(19-20) Tricyclazole (bekannt aus DE-A 22 50 077) der Formel

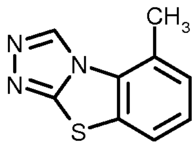

(19-21) N-({4-[(Cyclopropylamino)carbonyl]phenyl}sulfonyl)-2-methoxybenzamid der Formel

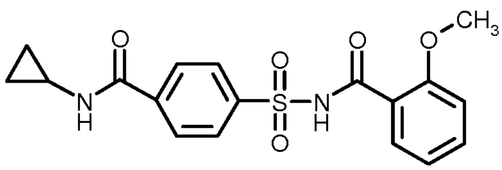

Bevorzugte Kombinationspartner der Gruppe (20) sind

(20-1) Pencycuron (bekannt aus DE-A 27 32 257) der Formel

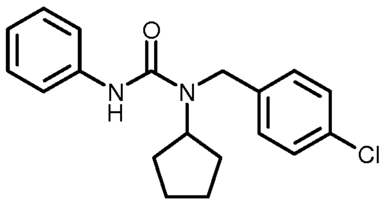

(20-2) Thiophanate-methyl (bekannt aus DE-A 18 06 123) der Formel

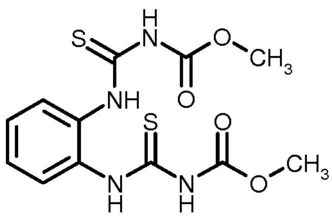

(20-3) Thiohanate-ethyl (bekannt aus DE-A 18 06 123) der Formel

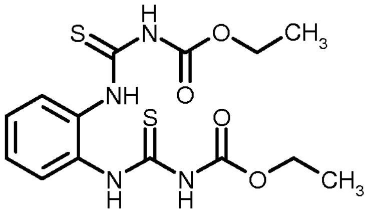

Bevorzugte Kombinationspartner der Gruppe (21) sind

(21-1) Fenoxanil (bekannt aus EP-A 0 262 393) der Formel

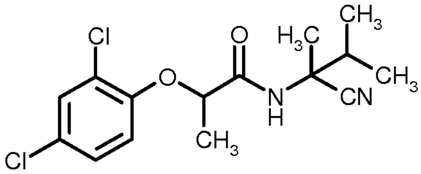

(21-2) Dicylcomat (bekannt aus JP-A 7-206608) der Formel

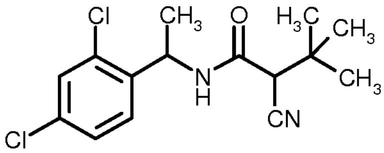

Bevorzugte Kombinationspartner der Gruppe (22) sind

(22-1) 5-Chlor-*N*-[*(1S)*-2,2,2-trifluor-1-methylethyl]-6-(2,4,6-trifluorphenyl)[1,2,4]triazolo[1,5-a]-pyrimidin-7-amin (bekannt aus US 5,986,135) der Formel

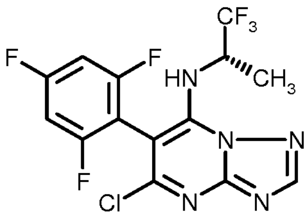

(22-2) 5-Chlor-*N*-[*(1R)*-1,2-dimethylpropyl]-6-(2,4,6-trifluorphenyl)[1,2,4]triazolo[1,5-a]pyrimidin-7-amin (bekannt aus WO 02/38565) der Formel

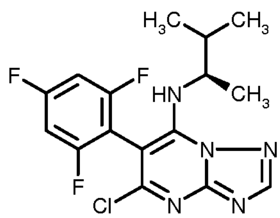

(22-3) 5-Chlor-6-(2-chlor-6-fluorphenyl)-7-(4-methylpiperidin-1-yl)[1,2,4]triazolo[1,5-a]pyrimidin (bekannt aus US 5,593,996) der Formel

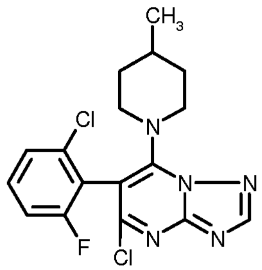

Bevorzugte Kombinationspartner der Gruppe (23) sind

(23-1) 2-Butoxy-6-iod-3-propyl-benzopyran-4-on (bekannt aus WO 03/014103) der Formel

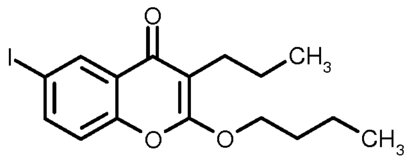

(23-2) 2-Ethoxy-6-iod-3-propyl-benzopyran-4-on (bekannt aus WO 03/014103) der Formel

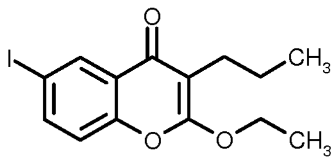

(23-3) 6-Iod-2-propoxy-3-propyl-benzopyran-4-on (bekannt aus WO 03/014103) der Formel

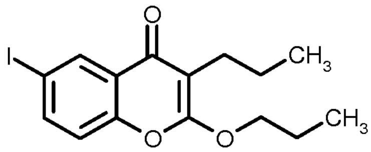

(23-4) 2-But-2-inyloxy-6-iod-3-propyl-benzopyran-4-on (bekannt aus WO 03/014103) der Formel

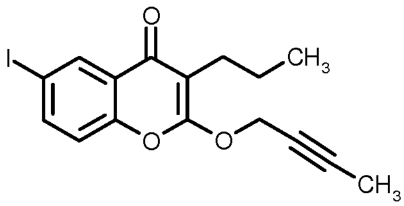

(23-5) 6-Iod-2-(1-methyl-butoxy)-3-propyl-benzopyran-4-on (bekannt aus WO 03/014103) der Formel

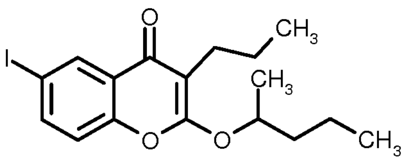

(23-6) 2-But-3-enyloxy-6-iod-benzopyran-4-on (bekannt aus WO 03/014103) der Formel

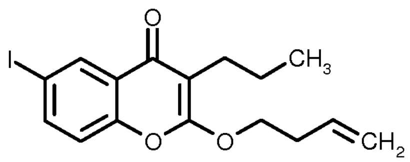

(23-7) 3-Butyl-6-iod-2-isopropoxy-benzopyran-4-on (bekannt aus WO 03/014103) der Formel

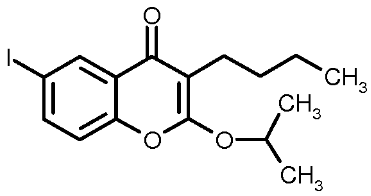

Bevorzugte Kombinationspartner der Gruppe (24) sind

(24-1) *N*-(3',4'-Dichlor-5-fluor-1,1'-biphenyl-2-yl)-3-(difluormethyl)-1-methyl-1*H*-pyrazol-4-carboxamid (bekannt aus WO 03/070705) der Formel

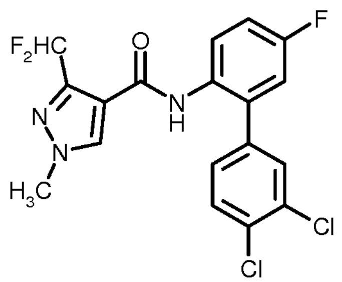

(24-2) 3-(Difluormethyl)-*N*-{3'-fluor-4'-[(*E*)-(methoxyimino)methyl]-1,1'-biphenyl-2-yl}-1-methyl-1*H*-pyrazol-4-carboxamid (bekannt aus WO 02/08197) der Formel

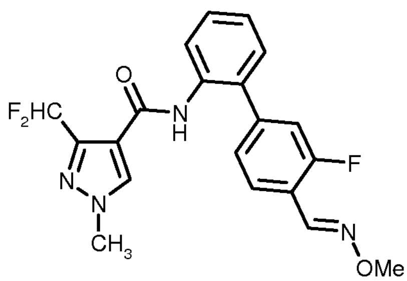

(24-3) 3-(Trifluormethyl)-*N*-{3'-fluor-4'-[(*E*)-(methoxyimino)methyl]-1,1'-biphenyl-2-yl}-1-methyl-1*H*-pyrazol-4-carboxamid (bekannt aus WO 02/08197) der Formel

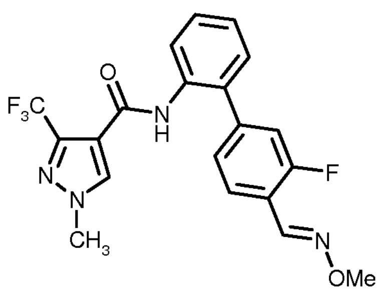

(24-4) *N*-(3',4'-Dichlor-1,1'-biphenyl-2-yl)-5-fluor-1,3-dimethyl-1*H*-pyrazol-4-carboxamid (bekannt aus WO 00/14701) der Formel

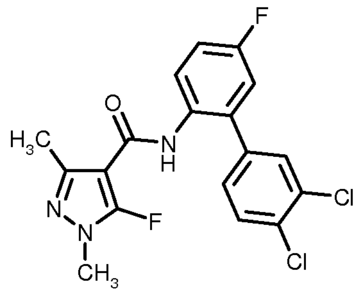

(24-5) *N*-(4'-Chlor-3'-fluor-1,1'-biphenyl-2-yl)-2-methyl-4-(trifluormethyl)-1,3-thiazol-5-carboxamid (bekannt aus WO 03/066609) der Formel

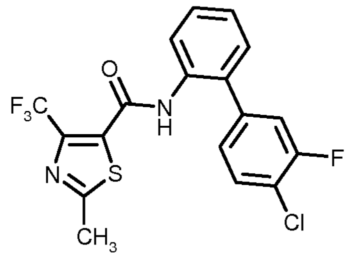

(24-6) *N*-(4'-Chlor-1,1'-biphenyl-2-yl)-4-(difluormethyl)-2-methyl-1,3-thiazol-5-carboxamid (bekannt aus WO 03/066610) der Formel

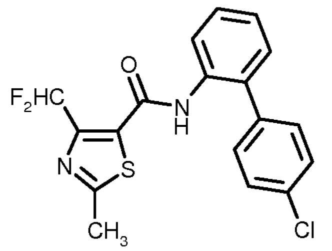

(24-7) *N*-(4'-Brom-1,1'-biphenyl-2-yl)-4-(difluormethyl)-2-methyl-1,3-thiazol-5-carboxamid (bekannt aus WO 03/066610) der Formel

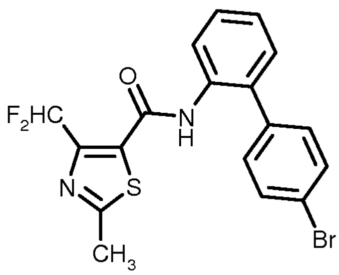

(24-8) 4-(Difluormethyl)-2-methyl-*N*-[4'-(trifluormethyl)-1,1'-biphenyl-2-yl]-1,3-thiazol-5-carboxamid (bekannt aus WO 03/066610) der Formel

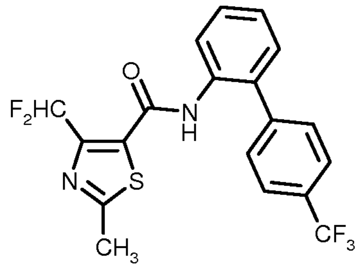

Die Verbindung (6-7) Carpropamid besitzt drei asymmetrische substituierte Kohlenstoffatome. Die Verbindung (6-7) kann daher als Gemisch von verschiedenen Isomeren oder auch in Form einer einzigen Komponente vorliegen. Besonders bevorzugt sind die Verbindungen

(1*S*,3*R*)-2,2-Dichlor-*N*-[(1*R*)-1-(4-chlorphenyl)ethyl]-1-ethyl-3-methylcyclopropancarboxamid der Formel

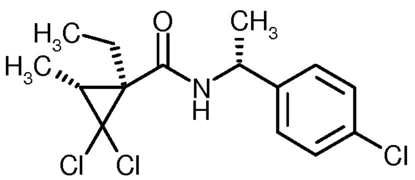

und

(1*R*,3*S*)-2,2-Dichlor-*N*-[(1*R*)-1-(4-chlorphenyl)ethyl]-1-ethyl-3-methylcyclopropancarboxamid der Formel

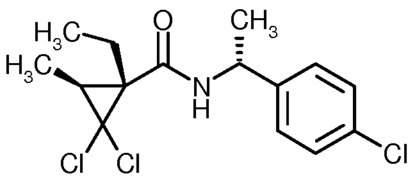

Die folgenden fungiziden Wirkstoffe sind besonders bevorzugt:

(2-1) Azoxystrobin
(2-2) Fluoxastrobin
(2-3) (2*E*)-2-(2-{[6-(3-Chlor-2-methylphenoxy)-5-fluor-4-pyrimidinyl]oxy}phenyl)-2-(methoxyimino)-N-methylethanamid
(2-4) Trifloxystrobin
(2-5) (2*E*)-2-(Methoxyimino)-*N*-methyl-2-(2-{[({(1*E*)-1-[3-(trifluormethyl)phenyl]ethyliden}amino)oxy]methyl}phenyl)ethanamid
(2-6) (2*E*)-2-(Methoxyimino)-*N*-methyl-2-{2-[(*E*)-({1-[3-(trifluormethyl)phenyl]-ethoxy}imino)methyl]phenyl}ethanamid
(2-8) 5-Methoxy-2-methyl-4-(2-{[({(1*E*)-1-[3-(trifluormethyl)phenyl]ethyliden}-amino)oxy]methyl}phenyl)-2,4-dihydro-3*H*-1,2,4-triazol-3-on
(2-11) Picoxystrobin
(2-9) Kresoxim-methyl
(2-10) Dimoxystrobin
(2-12) Pyraclostrobin
(2-13) Metominostrobin
(3-3) Propiconazole
(3-4) Difenoconazole
(3-6) Cyproconazole
(3-7) Hexaconazole
(3-8) Penconazole
(3-9) Myclobutanil
(3-10) Tetraconazole
(3-13) Flusilazole

(3-15) Prothioconazole
(3-16) Fenbuconazole
(3-17) Tebuconazole
(3-21) Bitertanol
(3-22) Triadimenol
(3-23) Triadimefon
(3-12) Epoxiconazole
(3-19) Metconazole
(3-24) Fluquinconazole
(4-1) Dichlofluanid
(4-2) Tolylfluanid
(5-1) Iprovalicarb
(5-3) Benthiavalicarb
(6-2) Boscalid
(6-5) Ethaboxam
(6-6) Fenhexamid
(6-7) Carpropamid
(6-8) 2-Chlor-4-[(2-fluor-2-methylpropanoyl)amino]-*N*,*N*-dimethylbenzamid
(6-9) Picobenzamid
(6-10) Zoxamide
(6-11) 3,4-Dichlor-N-(2-cyanophenyl)isothiazol-5-carboxamid
(6-14) Penthiopyrad
(6-16) *N*-[2-(1,3-Dimethylbutyl)phenyl]-1-methyl-4-(trifluormethyl)-1*H*-pyrrol-3-carboxamid
(7-1) Mancozeb
(7-2) Maneb
(7-4) Propineb
(7-5) Thiram
(7-6) Zineb
(8-1) Benalaxyl
(8-2) Furalaxyl
(8-3) Metalaxyl
(8-4) Metalaxyl-M
(8-5) Benalaxyl-M
(9-1) Cyprodinil
(9-2) Mepanipyrim
(9-3) Pyrimethanil
(10-1) 6-Chlor-5-[(3,5-dimethyfisoxazol-4-yl)sulfonyl]-2,2-difluor-5H-[1,3]dioxolo[4,5-f]-benzimidazol
(10-3) Carbendazim
(11-1) Diethofencarb
(11-2) Propamocarb
(11-3) Propamocarb-hydrochloride
(11-4) Propamocarb-Fosetyl
(12-2) Captan
(12-3) Folpet
(12-4) Iprodione
(12-5) Procymidone
(13-1) Dodine
(13-2) Guazatine
(13-3) Iminoctadine triacetate
(14-1) Cyazofamid
(14-2) Prochloraz
(14-3) Triazoxide
(15-5) Dimethomorph
(15-4) Fenpropimorph
(16-2) Fludioxonil
(17-1) Fosetyl-Al
(17-2) Phosphonic acid
(19-1) Acibenzolar-S-methyl

(19-2) Chlorothalonil
(19-3) Cymoxanil
(19-5) Famoxadone
(19-6) Fluazinam
(19-9) Oxadixyl
(19-10) Spiroxamine
(19-7) Kupferoxychlorid
(19-13) Fenamidone
(20-1) Pencycuron
(20-2) Thiophanate-methyl
(22-1) 5-Chlor-*N*-[*(1S)*-2,2,2-trifluor-1-methylethyl]-6-(2,4,6-trifluorphenyl)[1,2,4]triazolo[1,5-a]-pyrimidin-7-amin
(22-2) 5-Chlor-*N*-[*(1R)*-1,2-dimethylpropyl]-6-(2,4,6-trifluorphenyl)[1,2,4]triazolo[1,5-a]pyrimidin-7-amin
(23-1) 2-Butoxy-6-iod-3-propyl-benzopyran-4-on
(23-2) 2-Ethoxy-6-iod-3-propyl-benzopyran-4-on
(23-3) 6-Iod-2-propoxy-3-propyl-benzopyran-4-on
(24-1) *N*-(3',4'-Dichlor-5-fluor-1,1'-biphenyl-2-yl)-3-(difluormethyl)-1-methyl-1*H*-pyrazol-4-carboxamid
(24-3) 3-(Trifluormethyl)-*N*-{3'-fluor-4'-[(*E*)-(methoxyimino)methyl]-1,1'-biphenyl-2-yl}-1-methyl-1*H*-pyrazol-4-carboxamid
(24-7) *N*-(4'-Brom-1,1'-biphenyl-2-yl)-4-(difluormethyl)-2-methyl-1,3-thiazol-5-carboxamid

Weiterhin bevorzugt sind die folgenden Kombinationen:

Mefenpyr-diethyl und Prothioconazole,
Mefenpyr-diethyl und Fluoxastrobin,
Mefenpyr-diethyl und Trifloxystrobin,
Mefenpyr-diethyl und Tebuconazol.

jeweils in Kombination mit einer weiteren Verbindung aus den Gruppen 2 bis 24.

Auch eine Mischung mit anderen bekannten Wirkstoffen, wie Herbiziden oder mit Düngemitteln und Wachstumsregulatoren ist möglich.

Wenn die Safener und Fungizide in den erfindungsgemäßen Wirkstoffkombinationen in bestimmten Gewichtsverhältnissen vorhanden sind, zeigt sich der wirkungssteigernde (synergistische) Effekt besonders deutlich. Jedoch können die Gewichtsverhältnisse der Wirkstoffe in den Wirkstoffkombinationen in einem relativ großen Bereich variiert werden. Im Allgemeinen enthalten die erfindungsgemäßen Kombinationen Safener und ein Fungizid aus einer der Gruppen (2) bis (24) in den in der nachfolgenden Tabelle beispielhaft angegebenen Mischungsverhältnisse.

Die Mischungsverhältnisse basieren auf Gewichtsverhältnissen. Das Verhältnis ist zu verstehen als Safener : Mischpartner.

Tabelle 22: Mischungsverhältnisse

| Mischpartner | bevorzugtes Mischungsverhältnis | besonders bevorzugtes Mischungsverhältnis |
|---|---|---|
| Gruppe (2): Strobilurine | 50 : 1 bis 1 : 50 | 10 : 1 bis 1 : 20 |
| (3-15): Prothioconazole | 50 : 1 bis 1 : 50 | 10 : 1 bis 1 : 20 |

Das Mischungsverhältnis ist in jedem Fall so zu wählen, dass eine synergistische Mischung erhalten wird. Die Mischungsverhältnisse zwischen dem Safener und einer Verbindung aus einer der Gruppen (2) kann auch zwischen den einzelnen Verbindungen einer Gruppe variieren.

Die Verbindungen der Gruppe 1 und der Gruppen 2 und 3 können gleichzeitig, und zwar gemeinsam oder getrennt oder nacheinander aufgebracht werden, wobei die Reihenfolge bei getrennter Applikation im Allgemeinen keine Auswirkung auf den Bekämpfungserfolg hat.

Die erfindungsgemäßen Kombinationen besitzen sehr gute fungizide Eigenschaften und lassen sich zur Bekämpfung von phytopathogenen Pilzen, wie Plasmodiophoromycetes, Oomycetes, Chytridiomycetes, Zygomycetes, Ascomycetes, Basidiomycetes, Deuteromycetes usw. einsetzen.

Die erfindungsgemäßen Kombinationen eignen sich besonders gut zur Bekämpfung von Erysiphe graminis, Pyrenophora teres und Leptosphaeria nodorum.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;

Erkrankungen, hervorgerufen durch Erreger des Echten Mehltaus wie z.B.
Blumeria-Arten, wie beispielsweise Blumeria graminis;
Podosphaera-Arten, wie beispielsweise Podosphaera leucotricha;
Sphaerotheca-Arten, wie beispielsweise Sphaerotheca fuliginea;
Uncinula-Arten, wie beispielsweise Uncinula necator;
Erkrankungen, hervorgerufen durch Erreger von Rostkrankheiten wie z.B.
Gymnosporangium-Arten, wie beispielsweise Gymnosporangium sabinae
Hemileia-Arten, wie beispielsweise Hemileia vastatrix;
Phakopsora-Arten, wie beispielsweise Phakopsora pachyrhizi und Phakopsora meibomiae;
Puccinia-Arten, wie beispielsweise Puccinia recondita;
Uromyces-Arten, wie beispielsweise Uromyces appendiculatus;

Erkrankungen, hervorgerufen durch Erreger der Gruppe der Oomyceten wie z.B.
Bremia-Arten, wie beispielsweise Bremia lactucae;
Peronospora-Arten, wie beispielsweise Peronospora pisi oder P. brassicae;
Phytophthora-Arten, wie beispielsweise Phytophthora infestans;
Plasmopara-Arten, wie beispielsweise Plasmopara viticola;
Pseudoperonospora-Arten, wie beispielsweise Pseudoperonospora humuli oder Pseudoperonospora cubensis;
Pythium-Arten, wie beispielsweise Pythium ultimum;

Blattfleckenkrankheiten und Blattwelken, hervorgerufen durch z.B.
Alternaria-Arten, wie beispielsweise Alternaria solani;
Cercospora-Arten, wie beispielsweise Cercospora beticola;
Cladiosporum-Arten, wie beispielsweise Cladiosporium cucumerinum;
Cochliobolus-Arten, wie beispielsweise Cochliobolus sativus (Konidienform: Drechslera, Syn: Helminthosporium);
Colletotrichum-Arten, wie beispielsweise Colletotrichum lindemuthanium;
Cycloconium-Arten, wie beispielsweise Cycloconium oleaginum;
Diaporthe-Arten, wie beispielsweise Diaporthe citri;
Elsinoe-Arten, wie beispielsweise Elsinoe fawcettii;
Gloeosporium-Arten, wie beispielsweise Gloeosporium laeticolor;
Glomerella-Arten, wie beispielsweise Glomerella cingulata;
Guignardia-Arten, wie beispielsweise Guignardia bidwelli;
Leptosphaeria-Arten, wie beispielsweise Leptosphaeria maculans;
Magnaporthe-Arten, wie beispielsweise Magnaporthe grisea;
Mycosphaerella-Arten, wie beispielsweise Mycosphaerelle graminicola;
Phaeosphaeria-Arten, wie beispielsweise Phaeosphaeria nodorum;
Pyrenophora-Arten, wie beispielsweise Pyrenophora teres;
Ramularia-Arten, wie beispielsweise Ramularia collo-cygni;
Rhynchosporium-Arten, wie beispielsweise Rhynchosporium secalis;
Septoria-Arten, wie beispielsweise Septoria apii;
Typhula-Arten, wie beispielsweise Typhula incarnata;
Venturia-Arten, wie beispielsweise Venturia inaequalis;

Wurzel- und Stengelkrankheiten, hervorgerufen durch z.B.
Corticium-Arten, wie beispielsweise Corticium graminearum;
Fusarium-Arten, wie beispielsweise Fusarium oxysporum;
Gaeumannomyces-Arten, wie beispielsweise Gaeumannomyces graminis;
Rhizoctonia-Arten, wie beispielsweise Rhizoctonia solani;
Tapesia-Arten, wie beispielsweise Tapesia acuformis;
Thielaviopsis-Arten, wie beispielsweise Thielaviopsis basicola;

Ähren- und Rispenerkrankungen (inklusive Maiskolben), hervorgerufen durch z.B.

Alternaria-Arten, wie beispielsweise Alternaria spp.;
Aspergillus-Arten, wie beispielsweise Aspergillus flavus;
Cladosporium-Arten, wie beispielsweise Cladosporium spp.;
Claviceps-Arten, wie beispielsweise Claviceps purpurea;
Fusarium-Arten, wie beispielsweise Fusarium culmorum;
Gibberella-Arten, wie beispielsweise Gibberella zeae;
Monographella-Arten, wie beispielsweise Monographella nivalis;

Erkrankungen, hervorgerufen durch Brandpilze wie z.B.
Sphacelotheca-Arten, wie beispielsweise Sphacelotheca reiliana;
Tilletia-Arten, wie beispielsweise Tilletia caries;
Urocystis-Arten, wie beispielsweise Urocystis occulta;
Ustilago-Arten, wie beispielsweise Ustilago nuda;
Fruchtfäule hervorgerufen durch z.B.
Aspergillus-Arten, wie beispielsweise Aspergillus flavus;
Botrytis-Arten, wie beispielsweise Botrytis cinerea;
Penicillium-Arten, wie beispielsweise Penicillium expansum;
Sclerotinia-Arten, wie beispielsweise Sclerotinia sclerotiorum;
Verticilium-Arten, wie beispielsweise Verticilium alboatrum;

Samen- und bodenbürtige Fäulen und Welken, sowie Sämlingserkrankungen, hervorgerufen durch z.B.
Fusarium-Arten, wie beispielsweise Fusarium culmorum;
Phytophthora Arten, wie beispielsweise Phytophthora cactorum;
Pythium-Arten, wie beispielsweise Pythium ultimum;
Rhizoctonia-Arten, wie beispielsweise Rhizoctonia solani;
Sclerotium-Arten, wie beispielsweise Sclerotium rolfsii;

Krebserkrankungen, Gallen und Hexenbesen, hervorgerufen durch z.B.
Nectria-Arten, wie beispielsweise Nectria galligena;

Welkeerkrankungen hervorgerufen durch z.B.
Monilinia-Arten, wie beispielsweise Monilinia laxa;

Deformationen von Blättern, Blüten und Früchten, hervorgerufen durch z.B.
Taphrina-Arten, wie beispielsweise Taphrina deformans;

Degenerationserkrankungen holziger pflanzen, hervorgerufen durch z.B.
Esca-Arten, wie beispielsweise Phaemoniella clamydospora;

Blüten- und Samenerkrankungen, hervorgerufen durch z.B.
Botrytis-Arten, wie beispielsweise Botrytis cinerea;

Erkrankungen von Pflanzenknollen, hervorgerufen durch z.B.
Rhizoctonia-Arten, wie beispielsweise Rhizoctonia solani.

Die gute Pflanzenverträglichkeit der Kombination in den zur Bekämpfung von Pflanzenkrankheiten notwendigen Konzentrationen erlaubt eine Behandlung von ganzen Pflanzen (oberirdische Pflanzenteile und Wurzeln), von Pflanz- und Saatgut, und des Bodens. Die erfindungsgemäßen Kombinationen können zur Blattapplikation oder auch als Beizmittel eingesetzt werden.

Die gute Pflanzenverträglichkeit der verwendbaren Kombinationen in den zur Bekämpfung von Pflanzenkrankheiten notwendigen Konzentrationen erlaubt eine Behandlung des Saatguts. Die erfindungsgemäßen Kombinationen können somit als Beizmittel eingesetzt werden.

Ein großer Teil des durch phytopathogene Pilze verursachten Schadens an Kulturpflanzen entsteht bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von phytopathogenen Pilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von phytopathogenen Pilzen, indem das Saatgut mit einer erfindungsgemäßen Kombination behandelt wird.

Die Erfindung bezieht sich ebenfalls auf die Verwendung der erfindungsgemäßen Kombination zur Behandlung von Saatgut zum Schutz des Saatguts und der keimenden Pflanze vor phytopathogenen Pilzen.

Weiterhin bezieht sich die Erfindung auf Saatgut, welches zum Schutz vor phytopathogenen Pilzen mit einer erfindungsgemäßen Kombination behandelt, insbesondere beschichtet wurde.

Einer der Vorteile der Erfindung ist es, dass bei Verwendung von systemischen Fungiziden die Behandlung des Saatguts mit den erfindungsgemäßen Kombinationen nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Kombinationen insbesondere auch bei transgenem Saatgut eingesetzt werden können.

Die erfindungsgemäßen Kombinationen eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Im Rahmen der vorliegenden Erfindung wird die erfindungsgemäße Kombination alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge der auf das Saatgut aufgebrachten erfindungsgemäßen Kombination und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Kombinationen können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäßen Kombinationen eignen sich auch zur Steigerung des Ernteertrages. Sie sind außerdem mindertoxisch und weisen eine gute Pflanzenverträglichkeit auf.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die erfindungsgemäßen Kombinationen können in Abhängigkeit von ihren jeweiligen physikalischen und/oder chemischen Eigenschaften in die üblichen Formulierungen überführt werden, wie Lösungen, Emulsionen, Suspensionen, Pulver, Stäubemittel, Schäume, Pasten, lösliche Pulver, Granulate, Aerosole, Suspensions-Emulsions-Konzentrate, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe bzw. der Wirkstoffkombinationen mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid.

Als feste Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier- und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Der Wirkstoffgehalt der aus den handelsüblichen Formulierungen bereiteten Anwendungsformen kann in weiten Bereichen variieren. Die Wirkstoffkonzentration der Anwendungsformen zum Bekämpfen tierischer Schädlingen wie Insekten und Akariden kann von 0,0000001 bis zu 95 Gew.-% Wirkstoff, vorzugsweise zwischen 0,0001 und 1 Gew.-% liegen. Die Anwendung geschieht in einer den Anwendungsformen angepassten üblichen Weise.

Die Formulierungen zur Bekämpfung unerwünschter phytopathogener Pilze enthalten im Allgemeinen zwischen 0,1 und 95 Gew.-% Wirkstoffe, vorzugsweise zwischen 0,5 und 90 %.

Die erfindungsgemäßen Kombinationen können als solche, in Form ihrer Formulierungen oder den daraus bereiteten Anwendungsformen, wie gebrauchsfertige Lösungen, emulgierbare Konzentrate, Emulsionen, Suspensionen, Spritzpulver, lösliche Pulver, Stäubemittel und Granulate, angewendet werden. Die Anwendung geschieht in üblicher Weise, z.B. durch Gießen (drenchen), Tröpfchenbewässerung, Verspritzen, Versprühen, Verstreuen, Verstäuben, Verschäumen, Bestreiche, Verstreichen, Trockenbeizen, Feuchtbeizen, Nassbeizen, Schlämmbeizen, Inkrustieren usw.

Die erfindungsgemäßen Kombinationen können in handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen.

Die erfindungsgemäßen Kombinationen können gleichzeitig, und zwar gemeinsam oder getrennt, oder nacheinander aufgebracht werden, wobei die Reihenfolge bei getrennter Applikation im allgemeinen keine Auswirkung auf den Bekämpfungserfolg hat. In einer bevorzugten Ausführungsform wird die erfindungsgemäße Kombination gleichzeitig, vorzugsweise gemeinsam, aufgebracht.

In einer weiteren bevorzugten Ausführungsform wird die erfindungsgemäße Kombination nacheinander aufgebracht.

Beim Einsatz der erfindungsgemäßen Wirkstoffkombinationen können die Aufwandmengen je nach Applikationsart innerhalb eines größeren Bereichs variiert werden. Bei der Behandlung von Pflanzenteilen liegen die Aufwandmengen an Wirkstoffkombination im allgemeinen zwischen 0,1 und 10 000 g/ha, vorzugsweise zwischen 10 und 1000 g/ha. Bei der Saatgutbehandlung liegen die Aufwandmengen an Wirkstoffkombination im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoffkombination im allgemeinen zwischen 0,1 und 10 000 g/ha, vorzugsweise zwischen 1 und 5 000 g/ha.

Die Kombinationen können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die gute fungizide Wirkung der erfindungsgemäßen Kombinationen geht aus den nachfolgenden Beispielen hervor. Während die einzelnen Wirkstoffe in der fungiziden Wirkung Schwächen aufweisen, zeigen die Kombinationen Wirkung, obwohl die erfindungsgemäß verwendeten Safener in der Regel allein keine fungizide Wirkung aufweisen.

Ein synergistischer Effekt liegt bei Fungiziden immer dann vor, wenn die fungizide Wirkung der Wirkstoffkombinationen größer ist als die Summe der Wirkungen der einzeln applizierten Wirkstoffe.

Die zu erwartende fungizide Wirkung für eine gegebene Kombination zweier Wirkstoffe kann nach S.R. Colby ("Calculating Synergistic and Antagonistic Responses of Herbicide Combinations", Weeds 1967, 15, 20-22) wie folgt berechnet werden:

Wenn

X den *Wirkungsgrad* beim Einsatz des Wirkstoffes A in einer Aufwandmenge von *m g/ha* bedeutet,

Y den *Wirkungsgrad* beim Einsatz des Wirkstoffes B in einer Aufwandmenge von *n g/ha* bedeutet und

E den *Wirkungsgrad* beim Einsatz der Wirkstoffe A und B in Aufwandmengen von *m und n g/ha* bedeutet,

dann ist $$E = X + Y - \frac{X \times Y}{100}$$

Dabei wird der Wirkungsgrad in % ermittelt. Es bedeutet 0 % ein Wirkungsgrad, der demjenigen der Kontrolle entspricht, während ein Wirkungsgrad von 100 % bedeutet, dass kein Befall beobachtet wird.

Ist die tatsächliche fungizide Wirkung größer als berechnet, so ist die Kombination in ihrer Wirkung überadditiv, d.h. es liegt ein synergistischer Effekt vor. In diesem Fall muss der tatsächlich beobachtete Wirkungsgrad größer sein als der aus der oben angeführten Formel errechnete Wert für den erwarteten Wirkungsgrad (E).

Die Erfindung wird durch die folgenden Beispiele veranschaulicht. Die Erfindung ist jedoch nicht auf die Beispiele limitiert.

**Beispiel**

Erysiphe-Test (Weizen) / kurativ

Zur Herstellung einer zweckmäßigen Wirkstoffzubereitung verdünnte man eine handelsübliche Formulierung von Wirkstoff mit Wasser auf die gewünschte Konzentration.

Zur Prüfung auf kurative Wirksamkeit wurden junge Pflanzen mit Sporen von Erysiphe graminis f.sp. tritici bestäubt. 48 Stunden nach der Inokulation wurden die Pflanzen mit der Wirkstoffzubereitung in der angegebenen Aufwandmenge besprüht.

Die Pflanzen wurden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgte die Auswertung. Dabei bedeutet 0 % ein Wirkungsgrad, der demjenigen der Kontrolle entspricht, während ein Wirkungsgrad von 100 % bedeutet, daß kein Befall beobachtet wird.

**Tabelle**

| Erysiphe-Test (Weizen) / kurativ | | | |
|---|---|---|---|
| Wirkstoffe | Aufwandmenge an Wirkstoff in g/ha | Wirkungsgrad in % | |
| | | gef.* | ber.** |
| Mefenpyr | 25 | 0 | |
| Trifloxystrobin | 125 | 86 | |

(fortgesetzt)

| Erysiphe-Test (Weizen) / kurativ | | | |
|---|---|---|---|
| Wirkstoffe | Aufwandmenge an Wirkstoff in g/ha | Wirkungsgrad in % | |
| | | gef.* | ber.** |
| Fluoxastrobin | 125 | 0 | |
| Prothioconazole | 125 | 43 | |
| Mefenpyr + Trifloxystrobin 1:5 | 25 + 125 | 100 | 86 |
| Mefenpyr + Fluoxastrobin 1:5 | 25 + 125 | 100 | 0 |
| Mefenpyr + Prothioconazole 1:5 | 25 + 125 | 100 | 43 |
| * gef. = gefundene Wirkung<br>** ber. = nach der Colby-Formel berechnete Wirkung | | | |

## Patentansprüche

**1.** Fungizides Mittel, enthaltend

(a) ein Fungizid ausgewählt aus der Gruppe bestehend aus Trifloxystrobin, Fluoxastrobin und Prothioconazole, und einen Safener ausgewählt aus der Gruppe bestehend aus Mefenpyr-diethyl und Isoxadifen-ethyl,
oder
(b) ein Fungizid ausgewählt aus der Gruppe bestehend aus Azoxystrobin und Pyraclostrobin und einen Safener ausgewählt aus der Gruppe bestehend aus Mefenpyr-diethyl, Isoxadifen-ethyl, Cloquintocet-mexyl und N-[[4-[(cyclopropylamino)carbonyl]-phenyl]sulfonyl]-2-methoxybenzamid (= I-e-5),
oder
(c) das Fungizid Tebuconazole und den Safener Mefenpyr-diethyl.

**2.** Verwendung eines fungiziden Mittels gemäß Anspruch 1 zur Steigerung der mikrobiziden Wirkung des Fungizids.

**3.** Verfahren zum Bekämpfen von unerwünschten phytopathogenen Pilzen, **dadurch gekennzeichnet, dass** man ein fungizides Mittel gemäß Anspruch 1 auf die unerwünschten phytopathogenen Pilze und/oder deren Lebensraum und/oder das Saatgut von Pflanzensorten, die in der Landwirtschaft, im Gewächshaus, in Forsten oder im Gartenbau eingesetzt werden, ausbringt.

**4.** Verfahren gemäß Anspruch 3 **dadurch gekennzeichnet, dass** das Saatgut mit einem fungiziden Mittel gemäß Anspruch 1 gebeizt wird.

## Claims

**1.** Fungicidal composition, comprising

(a) a fungicide, selected from the group consisting of trifloxystrobin, fluoxastrobin and prothioconazole, and a safener selected from the group consisting of mefenpyr-diethyl and isoxadifen-ethyl,
or
(b) a fungicide, selected from the group consisting of azoxystrobin and pyraclostrobin, and a safener selected from the group consisting of mefenpyr-diethyl, isoxadifen-ethyl, cloquintocet-methyl and N-[[4-[(cyclopropylamine)carbonyl]-phenyl]sulfonyl]-2-methoxybenzamide (= I-e-5),
or
(c) the fungicide tebuconazole and the safener mefenpyr-diethyl.

**2.** Use of a fungicidal composition according to Claim 1 for increasing the microbicidal activity of the fungicide.

**3.** Method of controlling undesired phytopathogenic fungi, **characterized in that** the fungicidal composition according to Claim 1 is applied to the undesired phytopathogenic fungi and/or their environment and/or the seed of plant

varieties which are applied in agriculture, in the greenhouse, in forests or in horticulture.

**4.** Method according to Claim 3, **characterized in that** the seed is dressed with a fungicidal composition according to Claim 1.

## Revendications

**1.** Agent fongicide, contenant :

(a) un fongicide choisi dans le groupe constitué par la trifloxystrobine, la fluoxastrobine et le prothioconazole, et un agent protecteur choisi dans le groupe constitué par le méfenpyr-diéthyle et l'isoxadifène-éthyle,
ou
(b) un fongicide choisi dans le groupe constitué par l'azoxystrobine et la pyraclostrobine et un agent protecteur choisi dans le groupe constitué par le méfenpyr-diéthyle, l'isoxadifène-éthyle, le cloquintocet-mexyl et le N-[[4-[(cyclopropylamino)carbonyl]-phényl]sulfonyl]-2-méthoxybenzamide (= I-e-5),
ou
(c) le fongicide tébuconazole et l'agent protecteur méfenpyr-diéthyle.

**2.** Utilisation d'un agent fongicide selon la revendication 1 pour augmenter l'effet microbicide du fongicide.

**3.** Procédé de lutte contre des champignons phytopathogènes indésirables, **caractérisé en ce qu'**un agent fongicide selon la revendication 1 est appliqué sur les champignons phytopathogènes indésirables et/ou leur habitat et/ou les graines de variétés de plantes qui sont utilisées dans l'agriculture, dans les serres, dans les forêts ou dans l'horticulture.

**4.** Procédé selon la revendication 3, **caractérisé en ce que** les graines sont traitées avec un agent fongicide selon la revendication 1.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DD 277829
- JP 57112311 B
- JP 60123405 B
- EP 0382375 A
- DE 19602095 A
- DE 19646407 A
- EP 0712396 B
- EP 0460575 A
- EP 0569384 A
- EP 0596254 A
- DE 19539324 A
- WO 9823155 A
- EP 0253213 A
- EP 0398692 A
- EP 0278595 A
- DE 4423612 A
- DE 2551560 A
- EP 0112284 A
- EP 0258161 A
- DE 3406993 A
- DE 3042303 A
- DE 2735872 A
- EP 0145294 A
- EP 0234242 A
- EP 0015756 A
- EP 0196038 A
- EP 0068813 A
- EP 0537957 A
- WO 9616048 A
- DE 3721786 A
- EP 0040345 A
- EP 0329397 A
- EP 0378953 A
- DE 2324010 A
- DE 2201063 A
- EP 0183458 A
- DE 1193498 A
- DE 4026966 A
- WO 9604252 A
- EP 0256503 A
- DE 19531813 A
- EP 0315502 A
- EP 0737682 A
- EP 0639574 A
- EP 0339418 A
- EP 0341475 A
- EP 0600629 A
- WO 9942447 A
- EP 0604019 A
- WO 9924413 A
- US 3249499 A
- US 6616054 B
- WO 9618631 A
- WO 0238542 A
- DE 1234704 A
- US 2504404 A
- DE 1076434 A
- GB 935981 A
- US 1972961 A
- DE 1081446 A
- US 2588428 A
- DE 2903612 A
- DE 2513732 A
- DE 2515091 A
- WO 9601559 A
- EP 0310550 A
- EP 0270111 A
- DD 151404
- WO 9706171 A
- US 3631176 A
- US 3010968 A
- DE 1209799 A
- US 3206468 A
- EP 0078663 A
- US 3513241 A
- US 3178447 A
- US 2553770 A
- DE 2149923 A
- DE 2012656 A
- DE 2207576 A
- GB 1103989 A
- GB 1114155 A
- EP 0155509 A
- EP 0298196 A
- DE 2429523 A
- DE 2802488 A
- EP 0248086 A
- DD 140041
- GB 988630 A
- DE 2543279 A
- DE 2656747 A
- EP 0219756 A
- EP 0236272 A
- EP 0206999 A
- JP 6525876 B
- DE 2456627 A
- WO 9623793 A
- EP 0313512 A

- US 3290353 A
- DE 2312956 A
- DE 1493736 A
- EP 0393911 A
- EP 0031257 A
- DE 3030026 A
- DE 3735555 A
- JP 4429464 A
- EP 0897904 A
- EP 0629616 A
- WO 9914202 A
- US 3629428 A
- US 3856814 A
- GB 1094567 A
- JP 57055844 A
- EP 0019450 A
- DE 2250077 A
- DE 2732257 A
- DE 1806123 A
- EP 0262393 A
- JP 7206608 A
- US 5986135 A
- WO 0238565 A
- US 5593996 A
- WO 03014103 A
- WO 03070705 A
- WO 0208197 A
- WO 0014701 A
- WO 03066609 A
- WO 03066610 A
- US 4272417 A
- US 4245432 A
- US 4808430 A
- US 5876739 A
- US 20030176428 A1
- WO 2002080675 A1
- WO 2002028186 A2

**In der Beschreibung aufgeführte Nicht-Patentliteratur**

- The Pesticide Manual. 2003
- *Phytopatholy, Ecology and Epidemiology,* Dezember 1998, vol. 78 (12
- Apple spray programs used at East Malling to control scab and mildew. **BURCHILL, R.T. et al.** Report. East Malling Research Station, 1974, 161-5
- **S.R. COLBY.** Calculating Synergistic and Antagonistic Responses of Herbicide Combinations. *Weeds,* 1967, vol. 15, 20-22